# FASCICULE DE BREVET EUROPEEN

(11) **EP 2 037 955 B1**
(45) Date de publication et mention de la délivrance du brevet: **06.03.2019**
(21) Numéro de dépôt: 07788821.2
(22) Date de dépôt: 31.05.2007
(51) Int. Cl.: A61K 38/48

(54) **COMPOSITION DE FACTEUR VII RECOMBINANT OU TRANSGENIQUE, CHAQUE MOLECULE DE FACTEUR VII POSSEDANT DEUX SITES DE N-GLYCOSYLATION A MOTIFS GLYCANNIQUES DEFINIS**
REKOMBINANTER ODER TRANSGENER FAKTOR VII-ZUSAMMENSETZUNGEN, WOBEI JEDES FAKTOR VII-MOLEKÜL ZWEI N-GLYKOSYLIERUNGSSTELLEN MIT DEFINIERTEN GLYCAN-EINHEITEN ENTHÄLT
RECOMBINANT OR TRANSGENIC FACTOR VII COMPOSITION, EACH FACTOR VII MOLECULE HAVING TWO N-GLYCOSYLATION SITES WITH DEFINED GLYCAN UNITS

(30) Priorité: 31.05.2006 FR 0604872
(43) Date de publication de la demande: 25.03.2009
(73) Titulaire: Laboratoire Français du Fractionnement et des Biotechnologies, 91940 Les Ulis (FR)
(72) Inventeur: CHTOUROU, Abdessatar, Sami, F-78990 Elancourt (FR); NONY, Emmanuel, F-92160 Antony (FR); BIHOREAU, Nicolas, F-91400 Orsay (FR)
(74) Mandataire: IPAZ
(86) Numéro de dépôt international: PCT/FR2007/000909
(87) Numéro de publication internationale: WO 2007/138199

(56) Documents cités:
- EP-A1- 0 547 932
- EP-B1- 0 346 241
- WO-A2-02/29045
- WO-A2-2005/024006
- LUBON HENRYK ET AL: "VItamin K-dependent protein production in transgenic animals" THROMBOSIS AND HAEMOSTASIS, vol. 78, no. 1, 1997, pages 532-536, XP009079265 ISSN: 0340-6245
- KUMAR HARISH P M ET AL: "Elucidation of N-linked oligosaccharide structures of recombinant human factor VIII using fluorophore-assisted carbohydrate electrophoresis" BIOTECHNOLOGY AND APPLIED BIOCHEMISTRY, vol. 24, no. 3, 1996, pages 207-216, XP009079481 ISSN: 0885-4513
- DATABASE BIOSIS [Online] BIOSCIENCES INFORMATION SERVICE, PHILADELPHIA, PA, US; novembre 2004 (2004-11), ALJAMALI MAJED N ET AL: "Generation of transgenic mice expressing high levels of activated murine coagulation factor VII" XP002421223 Database accession no. PREV200510267900 & BLOOD, vol. 104, no. 11, Part 2, novembre 2004 (2004-11), page 398B, 46TH ANNUAL MEETING OF THE AMERICAN-SOCIETY-OF-HEMATOLOGY; SAN DIEGO, CA, USA; DECEMBER 04 -07, 2004 ISSN: 0006-4971
- SCHOENECKER J G ET AL: "Exposure to topical bovine thrombin during surgery elicits a response against the xenogeneic carbohydrate galactose alpha1-3galactose." JOURNAL OF CLINICAL IMMUNOLOGY NOV 2000, vol. 20, no. 6, novembre 2000 (2000-11), pages 434-444, XP002421224 ISSN: 0271-9142
- HIRONAKA T ET AL: "Comparative study of the sugar chains of factor VIII purified from human plasma and from the culture media of recombinant baby hamster kidney cells." THE JOURNAL OF BIOLOGICAL CHEMISTRY 25 APR 1992, vol. 267, no. 12, 25 avril 1992 (1992-04-25), pages 8012-8020, XP002421225 ISSN: 0021-9258 cité dans la demande

## Description

La présente invention se rapporte à un facteur VII recombinant ou transgénique obtenu sous forme d'une composition de facteur VII, chaque molécule de facteur VII possédant deux sites de N-glycosylation à motifs glycanniques définis, ainsi qu'à son utilisation comme médicament.

Le facteur VII (FVII) est une glycoprotéine vitamine K-dépendante qui, sous sa forme activée (FVIIa), participe au processus de coagulation en activant le facteur X et le facteur IX en présence de calcium et du facteur tissulaire. Le FVII est sécrété sous la forme d'une chaîne peptidique unique de 406 résidus, dont le poids moléculaire est d'environ 50 kDa. Le FVII comporte quatre domaines structuraux distincts : le domaine γ-carboxylique (Gla) N-terminal, deux domaines "epidermal growth factor (EGF)-like", ainsi qu'un domaine sérine protéase. L'activation du FVII en FVIIa est caractérisée par la coupure de la liaison Arg₁₅₂-Ile₁₅₃ (Arginine 152-Isoleucine 153). Le FVIIa est donc composé d'une chaîne légère de 152 acides aminés de poids moléculaire d'environ 20 kDa et d'une chaîne lourde de 254 acides aminés de poids moléculaire d'environ 30 kDa) liées entre elles par un seul pont disulfure (Cys₁₃₅-Cys₂₆₂).

Le FVIIa plasmatique comporte plusieurs modifications post-traductionnelles : les dix premiers acides glutamiques sont γ-carboxylés, l'Asp₆₃ est partiellement hydroxylé, la Ser₅₂ (Sérine 52) et la Ser₆₀ (Sérine 60) sont O-glycosylées et portent respectivement les motifs Glucose (Xylose)₀₋₂ et Fucose, l'Asn₁₄₅ (Asparagine 145) et l'Asn₃₂₂ (Asparagine 322) sont N-glycosylées majoritairement par des structures complexes biantennées bisialylées.

Le FVII est utilisé dans le traitement des patients atteints d'hémophilie, présentant un déficit en facteur VIII (hémophilie de type A) ou en facteur IX (hémophilie de type B), ainsi que des patients présentant d'autres déficiences des facteurs de coagulation, par exemple un déficit héréditaire en FVII. Il est donc nécessaire de disposer de concentrés de FVIIa injectables.

La méthode la plus ancienne d'obtention de concentrés de FVIIa a consisté en la purification du FVIIa à partir de protéines plasmatiques issues du fractionnement.

Le document EP 0 346 241 décrit, à cet effet, la préparation d'une fraction enrichie en FVIIa, obtenue après adsorption puis élution d'un sous-produit du fractionnement de protéines plasmatiques contenant le FVII et le FVIIa et d'autres protéines telles les facteurs IX (FIX), X (FX) et II (FII), notamment le prééluat du PPSB (P = prothrombine ou FII, P = proconvertine ou FVII, S = facteur de Stuart ou FX et B = facteur antihémophilique B ou FIX). L'inconvénient de ce procédé est que le FVII obtenu contient encore quelques traces d'autres facteurs de coagulation.

De même, le document EP 0 547 932 décrit un procédé de fabrication d'un concentré de FVIIa de haute pureté essentiellement dépourvu des facteurs vitamine K dépendants et du FVIII. Le FVII obtenu par ce procédé, malgré sa pureté, présente une activité thrombogénique résiduelle. <PAGE 2A>.

Ainsi, l'un des inconvénients majeurs de ces procédés est qu'ils ne permettent d'obtenir que de faibles quantités de produits. Par ailleurs, il demeure difficile d'obtenir un produit totalement dépourvu d'autres protéines présentes dans le plasma. Toutefois, les facteurs VII mentionnés dans les documents EP0346241 et EP0547932 sont des facteurs VII plasmatiques.

Enfin, bien que de nombreuses précautions soient prises à tous les stades de l'élaboration des facteurs plasmatiques de la coagulation pour assurer leur sécurité virale et bactérienne (suivis des donneurs de sang, tests pour détecter les contaminants viraux et bactériens connus, traitements rigoureux de purification et d'inactivation virale pour réduire au maximum le risque de transmission d'agents pathogènes véhiculés par le sang), tout risque de contamination par des agents pathogènes n'est pas néanmoins écarté. De plus, l'émergence de la nouvelle variante de la maladie de Creutzfeldt-Jakob a fait resurgir les craintes de transmission d'agents pathogènes non conventionnels par les produits issus du sang. En outre, le volume de plasma collecté auprès des donneurs de sang reste limité.

C'est pourquoi, dés les années 1980, l'ADN codant pour le facteur VII humain a été isolé (Hagen et al. (1986) ; Proc. Natl. Acad. Sci. USA ; Apr 83(8):2412-6) et exprimé dans les cellules de mammifères BHK (Baby Hamster Kidney) (document EP 0 200 421). Si cette méthode de production de FVII a pour avantage de contrôler le milieu dans lequel la protéine d'intérêt est produite, il est connu que les cellules de hamster confèrent aux protéines qu'elles expriment des motifs Galα1,3Gal, (Spiro RG et al, J. Biol. Chem, 1984, vol. 259, N° 15, 9858 et Furukawa K. et al, J. Biol. Chem, 1992, vol. 267, N° 12, 8012), dont l'immunogénicité est démontrée.

Il a été établi que 1% des lymphocytes B circulants chez l'homme produisent des anticorps dirigés contre l'épitote Galα1,3Gal (Galili et al, Blood, 1993, vol. 82, 2485). L'épitope et l'anticorps forment alors un complexe activant le complément et conduisant à des réactions immunitaires sévères, telles que les rejets aigus de greffes suite aux xenotransplantations. Il a été démontré que 15 à 20% des hémophiles traités à l'aide d'un FVII produit en cellule de hamster développent une réaction immunitaire (Prowse C.V et al, Blood Reviews, 1998, vol. 12, 99). Ce type de réaction immunitaire est dramatique dans le cas des hémophiles, car les FVII et FVIII devenus immunogènes vont entraîner des hémorragies très difficilement traitables.

Ainsi, il demeure nécessaire d'obtenir une composition de FVIIa recombinant ou transgénique dont l'immunogénicité est réduite, tout en étant la plus faible possible, et, de préférence, viralement sécurisé.

C'est pourquoi la Demanderesse a cherché à mettre au point une composition de FVII, de préférence viralement sécurisée, présentant une immunogénicité très réduite.

Ainsi, l'invention se rapporte à une composition de facteur VII recombinant ou transgénique, chaque molécule de facteur VII de la composition présentant deux sites de N-glycosylation, caractérisée en ce que parmi toutes les molécules de FVII de la composition, le taux de motifs glycanniques Galα1, 3Gal est compris entre 0 et 4% le taux de de motifs glycanniques Galα1,3Gal étant mesuré par lectine-blot avec révélation au 4-chloro-1-naphtol, et
- en ce que tous les acides sialiques du facteur VII impliquent des liaisons α2-6, et
- en ce que le facteur VII de la composition est produit par des lapines transgéniques.

La Demanderesse a trouvé de manière surprenante qu'un tel taux de motifs glycanniques Galα1, 3Gal dans le FVII de la composition de l'invention n'est pas immunogène lors de traitements de patients.

Le FVII de l'invention est sous forme d'une composition. En effet, tout FVII, qu'il soit plasmatique, recombinant ou transgénique, se présente sous forme d'un mélange de plusieurs protéines de FVII, ces protéines se différenciant entre elles notamment en ne présentant pas les mêmes modifications post-traductionnelles. Cette maturation post-traductionnelle est effectuée par les organites cellulaires au cours du transfert de la protéine de FVII entre les différents compartiments cellulaires. Ces modifications biochimiques modifient profondément la protéine, de telle sorte que la protéine finale est bien différente de la molécule directement codée par le gène. Ces modifications chimiques contribuent à la régulation de l'activité de la protéine, ainsi qu'à sa localisation. Ainsi, aux fins de l'invention, les expressions « FVII » et « composition de FVII » sont équivalentes.

Par « FVII recombinant ou transgénique », on désigne tout FVII issu du génie génétique et présentant les caractéristiques de modifications post-traductionnelles, en particulier de glycosylation énoncées, c'est-à-dire deux sites de N-glycosylation avec un taux nul ou très faible de Galα1, 3Gal dans la composition de FVII, ou encore assez faible pour ne pas être immunogène. Par opposition, le FVII de l'invention n'est pas un FVII plasmatique, c'est-à-dire qu'il n'est pas un produit purifié à partir de plasma humain ou animal.

Plus particulièrement, on désigne par FVII activé tout FVII activé issu du génie génétique présentant les caractéristiques de modifications post-traductionnelles précédentes, ainsi que deux sites de O-glycosylation à motifs glycanniques définis, une γ-carboxylation, et des ponts disulfures spécifiques.

Le motif Galα1, 3Gal est une structure composée de deux galactoses liés en α1,3. Il est situé à l'extrémité des antennes oligosaccharidiques des structures N-liées. Ce motif est connu pour son immunogénicité. En effet, ce motif glycannique est absent chez l'homme ainsi que chez certains singes, car le gène codant pour l'enzyme permettant sa synthèse (l'α1,3galactosyltransférase) a été inactivé. C'est pourquoi l'administration, chez l'homme, d'une protéine présentant un tel motif provoque l'apparition d'anticorps dirigés contre la protéine ainsi glycosylée.

Il est donc éminemment souhaitable de ne pas trouver un tel motif immunogène dans les protéines pharmaceutiques.

De préférence, la composition est caractérisé par une absence des motifs glycanniques Galα1, 3Gal dans toutes les molécules de facteur VII qui y sont présentes.

On entend ainsi désigner un FVII dont le taux de structures Galα1, 3Gal est nul ou si faible qu'il ne peut être distingué du bruit de fond obtenu par les mesures mises en oeuvre par les appareillages d'analyses actuellement disponibles, ou dont le taux n'est pas détectable notamment par une méthode de détection en lectine-blot avec révélation au 4-chloro-1-naphtol. Cette méthode de quantification est illustrée dans la partie « Exemples ». Cette expression désigne de manière équivalente tout FVII recombinant ou transgénique dont le taux de Galα1, 3Gal est proche de celui du FVII plasmatique. En tout état de cause, le taux de Galα1, 3Gal de la composition de FVII de l'invention n'est pas immunogène pour l'homme.

A l'inverse, un FVII recombinant disponible dans le commerce présente un taux de Galα1, 3Gal qui est détectable, qui peut donc être distingué du bruit de fond obtenu par les appareillages d'analyse.

Ainsi, selon la méthode de quantification utilisée, le motif Galα1, 3Gal pourra être soit totalement absent, soit présent à une taux inférieur à 4%, ou encore inférieur à 3,5%, ou encore à un taux inférieur à 3%, ce taux ne pouvant être distingué du bruit de fond. Avantageusement, le taux de motif Galα1, 3Gal est présent à un taux identique ou quasi-identique à celui du FVII plasmatique.

Le FVII de l'invention est un polypeptide dont la séquence peptidique peut être celle du FVII humain naturel, c'est-à-dire la séquence présente chez les humains ne présentant pas de troubles liés au FVII. Une telle séquence peut être codée par exemple par la séquence 1b décrite dans le document EP 0 200 421.

Avantageusement, la séquence du FVII de l'invention est la séquence SEQ ID NO : 1.

Dans un autre mode de réalisation, le FVII de l'invention peut être un variant du FVII humain naturel, dans la mesure où ce variant n'est pas plus immunogène que le FVII naturel. Ainsi, la séquence peptidique de ce variant peut posséder au moins 70% d'identité, et de manière avantageuse au moins 80% ou 90%, et de manière encore plus avantageuse au moins 99% d'identité avec la séquence du FVII humain naturel, un tel variant possédant essentiellement la même activité biologique que le FVII naturel.

Par ailleurs, le FVII de l'invention désigne également tout FVII dont l'activité biologique a été modifiée ou réduite par rapport à celle du FVII naturel. A titre d'exemple, on peut citer le FVII humain recombinant inactivé FFR-FVIIa, utilisé pour le traitement ou la prévention des thromboses (Holst et al, Eur.J.Vasc.Endovasc.Surg., 1998 Jun, 15(6) : 515-520). De tels FVII sont des polypeptides qui possèdent une séquence en acides aminés qui diffère de la séquence du FVII naturel par l'insertion, la délétion ou la substitution d'un ou de plusieurs acides aminés.

Enfin, dans un autre mode de réalisation, le FVII de l'invention peut être activé (FVIIa). Le FVIIa présente une activité coagulante 25 à 100 fois supérieure à celle du FVII lorsque ce dernier interagit en lieu et place du premier avec le facteur tissulaire (FT). L'activation du FVII résulte du clivage du zymogène par différentes protéases (FIXa, FXa, FVIIa) en deux chaînes réunies par un pont disulfure. Le FVIIa est le facteur de la coagulation responsable de l'hémostase chez les hémophiles ayant des anticorps circulants par exemple. D'une manière particulièrement avantageuse, le FVII de l'invention est totalement activé.

Le FVIIa de l'invention peut comporter plusieurs modifications post-traductionnelles : les neuf ou dix premiers acides glutamiques N-terminaux sont γ-carboxylés, l'Asp₆₃ est partiellement hydroxylé, la Ser₅₂ et la Ser₆₀ sont O-glycosylées et portent respectivement les motifs Glucose (Xylose)₀₋₂ et Fucose, l'Asn₁₄₅ et l'Asn₃₂₂ sont N-glycosylées majoritairement par des structures complexes biantennées monosialylées.

L'activité biologique du FVII de l'invention peut être quantifiée en mesurant la capacité d'une composition de FVII à induire la coagulation sanguine au moyen d'un plasma déficient en FVII et de la thromboplastine, comme par exemple décrit dans le brevet US 5,997,864. Dans le test décrit dans ce brevet, l'activité biologique est exprimée par une réduction du temps de coagulation par rapport à l'échantillon contrôle, et est convertie en « unités de FVII » par comparaison avec un standard de sérum humain (pool) contenant 1 unité/ml d'activité de FVII.

Le FVII recombinant de l'invention peut être obtenu au moyen des techniques standard, bien connues de l'homme du métier, permettant l'expression d'une protéine dans un système biologique.

Le FVII de l'invention peut être exprimé dans tout micro-organisme, plante ou animal conférant les caractéristiques de glycosylation énoncées, c'est-à-dire un taux très faible ou nul de Galα1, 3Gal dans la composition de FVII. Par micro-organisme, on entend tout système bactérien, fongique, viral ou cellulaire, les cellules pouvant être des cellules de plantes ou de mammifères. Les cellules de mammifères peuvent être des cellules animales ou humaines. Il peut s'agir également de toute cellule KnockOut pour l'α1,3galactosyltransférase.

Le FVII de l'invention peut également être produit dans des animaux ou des plantes transgéniques, dans la mesure où ces animaux ou plantes confèrent au FVII ou à la composition de FVII les caractéristiques de glycosylation énoncées, c'est-à-dire une absence ou un très faible taux de Galα1, 3Gal dans la composition de FVII. Les animaux peuvent être le lapin, le porc, le mouton, la chèvre, le boeuf, la poule, ou tout animal KnockOut pour l'α1,3galactosyltransférase, cette liste n'étant pas limitative.

Le FVII de l'invention comporte, comme le FVII humain, deux sites de N-glycosylation, en position 145 et 322, et 2 sites de O-glycosylation, en position 52 et 60. Dans un site de N-glycosylation, les chaînes d'oligosaccharides sont liées à une asparagine (N-liées). Dans un site de O-glycosylation, les chaînes d'oligosaccharides sont liées à une sérine. Les motifs liés sur ces acides aminés seront différents pour chaque protéine de la composition. Toutefois, on peut quantifier, pour la totalité de la composition, le taux de chaque motif glycannique, ou encore de chaque sucre.

Les pourcentages des différents glycannes donnés dans la présente demande ne tiennent pas compte de la O-glycosylation.

De préférence, la composition de FVII est caractérisée en ce que, parmi tous les motifs glycanniques du FVII de la composition, au moins 40% sont des formes glycanniques biantennées, monosialylées. Dans un autre mode de réalisation, les formes monosialylées sont présentes à au moins 50%. Dans un autre mode de réalisation, les formes monosialylées sont présentes à au moins 60%.

Avantageusement, les formes glycanniques biantennées, monosialylées du FVII sont majoritaires.

La composition de FVII se caractérise en ce que au moins certains des acides sialiques du facteur VII impliquent des liaisons α2-6.

Avantageusement, au moins 65% des acides sialiques du FVII impliquent des liaisons α2,6. De façon très avantageuse, au moins 70%, voire 80% et, en particulier, au moins 90% des acides sialiques du FVII impliquent des liaisons α2,6.

De façon particulièrement préférée, tous les acides sialiques impliquent des liaisons α2,6, c'est-à-dire que tous les acides sialiques sont liés au galactose par une liaison α2,6.

Le fait qu'au moins 65% des acides sialiques du FVII de la composition impliquent des branchements α2,6 est un des avantages du FVII de l'invention. En effet, les acides sialiques du FVII recombinant disponible dans le commerce impliquent uniquement des liaisons α2,3. Or, le FVII plasmatique est un mélange de ces deux isomères. Toutefois, ce dernier comporte plus de liaisons α2,6, ce qui rapproche encore le FVII de l'invention du FVII plasmatique.

Selon des modes de réalisation de l'invention, de 65% à 100% des acides sialiques du FVII impliquent des liaisons α2,6. De façon plus préférée, de 70% ou 80% à 100% des acides sialiques du FVII impliquent des liaisons α2,6.

Avantageusement, parmi les formes glycanniques biantennées monosialylées du FVII, les formes glycanniques majoritaires sont non fucosylées.

De préférence, ces formes glycanniques biantennées monosialylées non fucosylées sont présentes dans le FVII de la composition de l'invention à un taux supérieur à 20%. Avantageusement, ce taux est supérieur à 25%, ou encore supérieur à 40%.

D'une manière particulièrement avantageuse, le taux de fucosylation de la composition de FVII de l'invention est compris entre 20% et 50%. Dans un mode de réalisation de l'invention, ce taux peut être inférieur à 15%.

Cette caractéristique est l'un des avantages du FVII de l'invention. En effet, le FVII recombinant disponible dans le commerce présente un taux de fucosylation de 100%, alors que le FVII plasmatique possède un taux de fucosylation de 16% environ. Ainsi, la fucosylation du FVII de l'invention est proche de celle du FVII plasmatique, ce qui confère un avantage au FVII de l'invention en terme d'immunogénicité.

Avantageusement, le FVII de l'invention est un FVII transgénique. Ainsi, le FVII de l'invention est avantageusement un FVII recombinant produit de manière transgénique.

Dans un mode de réalisation particulier de l'invention, le FVII transgénique de l'invention est produit dans le lait d'un animal transgénique.

Une telle production de protéines peut être réalisée en greffant le gène codant pour la protéine d'intérêt sur la région régulatrice d'un des gènes de synthèse de protéines du lait qui va diriger celle-ci spécifiquement dans la glande mammaire puis sa sécrétion dans le lait.

De manière particulièrement avantageuse, le FVII de l'invention est produit par des lapines transgéniques.

Cette espèce est particulièrement avantageuse car le lapin ne semble pas sensible aux prions, notamment à l'encéphalopathie spongiforme subaiguë transmissible, qui constitue un problème de santé publique majeur.

De plus, la barrière d'espèce entre le lapin et l'homme est importante. A l'inverse, la barrière d'espèce entre l'homme et le hamster, qui est le système biologique dans lequel est produit le FVII recombinant disponible dans le commerce, est moins importante.

Ainsi, la production du FVII dans le lapin est avantageuse en termes de sécurité quant à la transmission d'agents pathogènes.

Dans un mode de réalisation préféré de l'invention, le FVII de l'invention est produit dans les glandes mammaires de lapines.

La sécrétion par les glandes mammaires de la protéine d'intérêt, permettant sa sécrétion dans le lait du mammifère transgénique est une technique bien connue de l'homme du métier, qui implique le contrôle de l'expression de la protéine recombinante de manière tissus-dépendante.

Le contrôle tissulaire de l'expression est effectué grâce à des séquences permettant l'expression de la protéine vers un tissu particulier de l'animal. Ces séquences sont notamment les séquences promoteur, ainsi que les séquences peptides signal.

Des exemples de promoteurs permettant l'expression d'une protéine d'intérêt dans les glandes mammaires sont le promoteur WAP (whey acidic protein), le promoteur de la caséine, tel que le promoteur de la β-caséine, le promoteur de la β-lactoglobuline, cette liste n'étant pas limitative.

Une méthode de production d'une protéine recombinante dans le lait d'un animal transgénique peut comporter les étapes suivantes : une molécule d'ADN synthétique comprenant un gène codant pour le FVII humain, ce gène étant sous le contrôle d'un promoteur d'une protéine sécrétée naturellement dans le lait, est intégrée dans un embryon d'un mammifère non humain. L'embryon est ensuite placé dans une femelle de mammifère de la même espèce. Une fois que le mammifère issu de l'embryon s'est suffisamment développé, la lactation du mammifère est induite, puis le lait collecté. Le lait contient alors le FVII transgénique d'intérêt.

Un exemple de procédé de préparation de protéine dans le lait d'une femelle de mammifère autre que l'être humain est donné dans le document EP 0 527 063, dont l'enseignement peut être repris pour la production de la protéine d'intérêt de l'invention, cet exemple n'étant pas limitatif.

Un plasmide contenant le promoteur WAP est fabriqué par introduction d'une séquence comportant le promoteur du gène WAP, ce plasmide étant réalisé de manière à pouvoir recevoir un gène étranger placé sous la dépendance du promoteur WAP. Le gène codant pour le FVII humain est intégré, et placé sous la dépendance du promoteur WAP. Le plasmide contenant le promoteur et le gène codant pour la protéine d'intérêt sont utilisés pour obtenir des lapines transgéniques, par microinjection dans le pronucléus mâle d'embryons de lapins. Les embryons sont ensuite transférés dans l'oviducte de femelles hormonalement préparées. La présence des transgènes est révélée par la technique de Southern à partir de l'ADN extrait des lapereaux transgéniques obtenus. Les concentrations dans le lait des animaux sont évaluées à l'aide de tests radioimmunologiques spécifiques.

Le FVII transgénique produit par la lapine dans son lait est obtenu sous forme d'une composition où chaque molécule de facteur VII de la composition présente deux sites de N-glycosylation, caractérisé en ce que parmi toutes les molécules de FVII de la composition, le taux de motifs glycanniques Galα1, 3Gal est inférieur à 4%, voire nul. Ainsi, de manière avantageuse, le FVII transgénique produit par la lapine ne possède pas de motif glycannique Galα1,3Gal.

De préférence, la composition de FVII transgénique de lapine de l'invention est caractérisée en ce que, parmi tous les motifs glycanniques de la composition, au moins 40% sont des formes glycanniques biantennées, monosialylées. Dans un autre mode de réalisation, les formes monosialylées sont présentes à au moins 50%. Dans un autre mode de réalisation, les formes monosialylées sont présentes à au moins 60%.

Avantageusement, les formes glycanniques biantennées, monosialylées sont majoritaires.

La composition de FVII se caractérise en ce que au moins certains des acides sialiques du facteur VII impliquent des liaisons α2-6.

Avantageusement, au moins 65% des acides sialiques du FVII impliquent des liaisons α2,6. De façon très avantageuse, au moins 70%, voire 80% et, en particulier, au moins 90% des acides sialiques du FVII impliquent des liaisons α2,6.

De façon particulièrement préférée, tous les acides sialiques impliquent des liaisons α2,6, c'est-à-dire que tous les acides sialiques sont liés au galactose par une liaison α2,6.

De préférence, parmi les formes glycanniques biantennées, monosialylées, les formes glycanniques majoritaires sont non fucosylées. Avantageusement, ces formes glycanniques biantennées, monosialylées non fucosylées sont présentes dans le FVII de cette composition à un taux supérieur à 20%. Avantageusement, ce taux est supérieur à 25%, ou encore supérieur à 40%.

De préférence, le taux de fucosylation du FVII de cette composition de l'invention est compris entre 20% et 50%. Dans un mode de réalisation de l'invention, ce taux peut être inférieur à 15%.

Le FVIIa transgénique de l'invention peut comporter plusieurs modifications post-traductionnelles : les neuf ou dix premiers acides glutamiques N-terminaux sont γ-carboxylés, l'Asp₆₃ (Asparagine 63) est partiellement hydroxylée, la Ser₅₂ (Sérine 52) et la Ser₆₀ (Sérine 60) sont O-glycosylées et portent respectivement les motifs Glucose (Xylose)₀₋₂ et Fucose, l'Asn₁₄₅ et l'Asn₃₂₂ sont N-glycosylées majoritairement par des structures complexes biantennées monosialylées.

Le FVII de l'invention peut être purifié à partir du lait par des techniques connues de l'homme du métier. Par exemple, une méthode de purification d'une protéine d'intérêt à partir de lait, telle que décrite dans le brevet US 6,268,487, peut comprendre les étapes suivantes consistant à : a) soumettre le lait à une filtration tangentielle sur une membrane de porosité suffisante pour former un rétentat et un perméat, le perméat contenant la protéine exogène, b) soumettre le perméat à un appareil de capture par chromatographie de manière à déplacer la protéine exogène et obtenir un effluent, c) combiner l'effluent et le rétentat, d) répéter les étapes a) à c) jusqu'à ce que le FVII soit séparé des lipides, des micelles de caséines, et que le FVII soit récupéré à au moins 75%.

Avantageusement, le FVII de l'invention est activé. Le FVIIa résulte, *in vivo*, du clivage du zymogène par différentes protéases (FIXa, FXa, FVIIa) en deux chaînes réunies par un pont disulfure. Le FVIIa seul a très peu d'activité enzymatique, mais complexé avec son cofacteur, le facteur tissulaire (FT), il déclenche le processus de la coagulation en activant le FX et le FIX.

Le FVIIa de l'invention est donc composé d'une chaîne légère de 152 acides aminés de poids moléculaire d'environ 20 kDa et d'une chaîne lourde de 254 acides aminés de poids moléculaire d'environ 30 kDa liées entre elles par un seul pont disulfure (Cys₁₃₅-Cys₂₆₂).

Ainsi, le FVII de l'invention est un FVII activé possédant une activité et une structure proche du FVII plasmatique.

Le FVIIa présente une activité coagulante 25 à 100 fois supérieure à celle du FVII lorsqu'ils interagissent avec le facteur tissulaire (FT).

Dans un mode de réalisation de l'invention, le FVII peut être activé *in vitro* par les facteurs Xa, VIIa, IIa, IXa et XIIa.

Le FVII de l'invention peut également être activé pendant son procédé de purification.

La Demanderesse a constaté de manière surprenante que le FVII, même placé sous le contrôle d'un promoteur d'une protéine naturellement produite dans le lactosérum, comme le promoteur WAP par exemple, est néanmoins susceptible de se trouver associé aux ions calcium du lait, et donc aux micelles de caséines.

Ainsi, il sera très avantageux de mettre au point un procédé d'extraction et de purification du FVII, susceptible de capter cette protéine qu'elle soit associée dans des complexes du lactosérun ou engagée dans des micelles de caséines, le FVII présentant une affinité pour les ions calcium du lait.

Par exemple, un procédé d'extraction et de purification de FVII transgénique, contenu dans du lait d'un animal transgénique, comprend les étapes suivantes consistant à :
a) extraire le FVII à partir du lait, le facteur VII étant lié aux sels et/ou complexes organiques et/ou inorganiques de calcium dudit lait, par la précipitation de composés de calcium obtenue par ajout dans le lait d'un sel soluble, dont l'anion est choisi pour son aptitude à former lesdits composés de calcium insolubles pour ainsi libérer le facteur VII desdits sels et/ou complexes, le facteur VII étant présent dans une phase liquide,
b) séparer la phase liquide enrichie en la protéine du précipité de composés de calcium, ladite phase liquide étant en outre séparée en une phase lipidique et une phase aqueuse non lipidique comprenant la protéine,
c) soumettre la phase aqueuse non lipidique à une étape de chromatographie d'affinité, en utilisant un tampon d'élution à base d'un sel de phosphate à une concentration prédéterminée, et
d) soumettre l'éluat de facteur VII, obtenu selon l'étape c), à deux ou trois étapes de chromatographie sur des colonnes échangeuses d'anions de type base faible utilisant des tampons appropriés pour des élutions successives du facteur VII retenu sur lesdites colonnes.

La Demanderesse a constaté de manière surprenante qu'un tel procédé faisait intervenir une étape d'extraction du FVII lié aux sels et/ou complexes organiques et/ou inorganiques de calcium dudit lait par précipitation de composés insolubles de calcium obtenus par ajout d'un sel soluble dans le lait, dont l'anion est choisi pour sa capacité à former des précipités de composés de calcium, alors que le FVII est libéré de ces sels et/ou complexes organiques et/ou inorganiques de calcium dudit lait et se retrouve en solution dans la phase liquide.

Le FVII est lié aux sels et/ou complexes organiques et/ou inorganiques de calcium dudit lait, ce qui signifie que le FVII présente une affinité pour ces sels et/ou complexes de calcium. Le FVII présente donc un nombre suffisant de sites de fixation à ces sels et/ou complexes pour y être lié, totalement ou partiellement.

Les sels et/ou complexes organiques et/ou inorganiques de calcium du lait représentent les sels phosphocalciques en interaction avec les micelles colloïdales de caséines. Les différentes caséines forment un complexe micellaire colloïdal, pouvant atteindre des diamètres d'environ 0,5 µm, avec des sels phosphocalciques, se présentant par exemple sous la forme d'agrégats (« clusters ») de phosphate tricalcique, soit Ca₉(PO₄)₆. De telles micelles sont formées de sous-unités de caséines constituées d'une couche hydrophile riche en caséine-κ entourant un noyau hydrophobe, les sels phosphocalciques étant liés par interaction électrostatique sur la couche hydrophile. Ces sels phosphocalciques peuvent également être présents dans le volume interne de la micelle sans être liés à la caséine. Ces sels et/ou complexes sont également présents dans le lait sous forme de phosphate monocalcique et/ou de phosphate dicalcique, qui sont en équilibre avec les autres formes ioniques de calcium en fonction des réactions chimiques et biochimiques mises en oeuvre. Ces sels et/ou complexes de calcium peuvent être présents dans le volume interne de la micelle de caséine.

Une telle affinité du FVII pour les sels et/ou complexes organiques et/ou inorganiques de calcium peut résulter des interactions de la protéine non modifiée ou modifiée in vivo ou in vitro par exemple par des modifications post-traductionnelles.

Dans le cadre du procédé de l'invention, le FVII transgénique représente tout FVII transgénique présentant des caractéristiques notamment de glycosylation très diverses. Il peut s'agir avantageusement du FVII de l'invention, tel que défini plus haut.

On entend par composés de calcium insolubles, des sels ou complexes de calcium dont la solubilité dans le lait est inférieure à 0,5%.

Le FVII est majoritairement associé aux sels phosphocalciques des micelles de caséines. Le FVII présente une forte affinité pour le calcium, dont la constante d'affinité est élevée, c'est-à-dire qu'au moins 70% jusqu'à 90% du FVII étant piégés dans les micelles de caséine. Le reste du FVII présente une affinité pour les autres formes les sels et/ou complexes organiques et/ou inorganiques de calcium mentionnés plus haut.

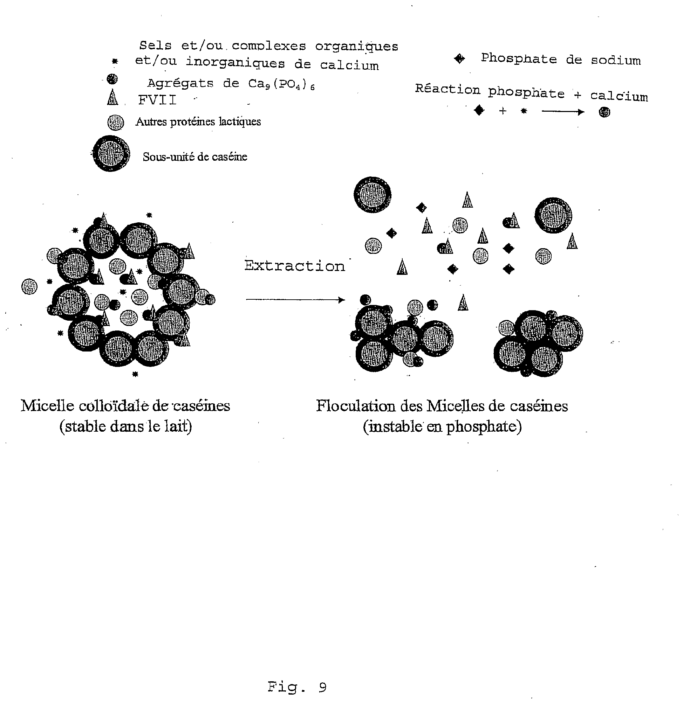

Sans être liée par une quelconque interprétation des mécanismes observés, la Demanderesse suppose que l'ajout du sel soluble déplace l'équilibre des sels phosphocalciques des micelles, provoquant ainsi leur déstructuration et la précipitation d'agrégats de sous-unités de caséines. Le FVII associé aux sels phosphocalciques piégé dans et/ou sur les micelles est libéré dans le milieu lors de cette déstructuration. De plus, le FVII est alors également libéré ou dissocié des sels phosphocalciques, car ceux-ci précipitent sous la forme de composés de calcium insolubles sous l'effet du sel soluble utilisé dans le procédé de l'invention. De même, le FVII associé aux sels et/ou complexes organiques et/ou inorganiques de calcium solubles en serait également dissocié, par le même type de réaction (voir figure 9).

Dans le cadre de l'invention, le sel soluble représente tout sel permettant d'obtenir l'effet voulu.

Le sel soluble utilisé dans le procédé de l'invention peut être présent dans le lait à une concentration choisie par l'homme du métier pour parvenir à la séparation du FVII à partir des sels et/ou complexes de calcium. A ce titre, il s'agit d'une concentration suffisante pour permettre la séparation d'au moins 20%, ou avantageusement d'au moins 30% jusqu'à 50% du FVII. De façon particulièrement avantageuse, il s'agit d'une concentration suffisante pour permettre la séparation d'au moins 60% jusqu'à 80%, ou d'au moins 90% du FVII.

Le procédé de l'invention permet la précipitation notamment des agrégats de sous-unités de caséines. Cette précipitation est due à la déstructuration des micelles de caséines, comme indiqué plus haut. La mise en oeuvre du procédé de l'invention déstabilise, par la précipitation, l'état colloïdal dans lequel se trouve le lait.

Le procédé de l'invention est donc un procédé permettant le passage du lait d'un état colloïdal à un état liquide, ce qui correspond à une extraction directe colloïdes/liquides.

Le procédé de l'invention permet également d'obtenir le lactosérum dont la coloration est plus claire que celle du lait de départ. En effet, ce sont les caséines qui donnent leur couleur blanche au lait. Une fois précipitées, elles ne peuvent plus conférer cette couleur au lait.

Par sel soluble selon l'invention on entend d'un sel dont la solubilité dans le lait est d'au moins 0,5 partie de sel par partie de lait (p/p).

Avantageusement, le sel soluble, utilisé dans le procédé, est un sel de phosphate. Le sel peut être dans une solution aqueuse qui est ensuite ajoutée au lait, ou il peut être ajouté directement au lait sous forme de poudre.

De préférence, le sel de phosphate est choisi dans le groupe constitué par le phosphate de sodium, le phosphate de lithium, le phosphate de potassium, le phosphate de rubidium et le phosphate de césium, et est, en particulier, le phosphate de sodium.

En variante, le sel soluble utilisé pour la mise en oeuvre du procédé de l'invention peut être un oxalate d'un métal alcalin, en particulier l'oxalate de sodium ou de potassium, ou un carbonate d'un métal alcalin, en particulier, le carbonate de sodium ou de potassium, ou leur mélange.

Avantageusement, la concentration du sel soluble en solution aqueuse, qui est ainsi préparée pour la mise en oeuvre du procédé, est comprise entre 100 mM et 3 M, de façon plus préférée, entre 200 mM et 500 mM et, en particulier, entre 200 mM et 300 mM.

Le lait dans lequel se trouve le FVII à extraire peut être du lait brut non écrémé ou du lait écrémé. L'avantage d'appliquer le procédé de l'invention à du lait écrémé réside dans le fait qu'il contient une quantité plus faible de lipides. Le procédé peut aussi être appliqué à du lait frais ou congelé.

Le procédé comprend une étape b) de séparation de la phase liquide en une phase lipidique et une phase aqueuse non lipidique comprenant la protéine qui est de préférence effectuée par centrifugation. La phase aqueuse non lipidique est en fait le lactosérum. Cette étape de séparation permet également d'isoler les agrégats de sous-unités micellaires de caséines et le précipité de composés de calcium.

Le procédé peut en outre comprendre, après l'étape de séparation, une étape de filtration de la phase aqueuse non lipidique effectuée successivement sur des filtres de porosité décroissante, de préférence, de 1 µm puis de 0,45 µm. L'utilisation de ces filtres, tels qu'à base de fibres de verre, permet de réduire la teneur en lipides, globules gras et de phospholipides naturellement présents dans le lait. Une porosité inférieure à 0,5 µm des filtres permet de maintenir la qualité bactériologique de la phase aqueuse non lipidique, ainsi que des supports de purification mis en oeuvre postérieurement (ultrafiltres, colonne de chromatographie etc.) (voir plus loin). La phase lipidique est, de préférence, filtrée à travers ces filtres qui retiennent ainsi complètement les globules lipidiques du lait, et le filtrat est clair.

Elle peut être suivie d'une étape de concentration/dialyse par ultrafiltration.

La concentration permet de réduire le volume de la phase aqueuse non lipidique en vue de sa conservation. La membrane d'ultrafiltration est choisie par l'homme du métier en fonction des caractéristiques de la protéine d'intérêt. De façon générale, un seuil de coupure dont la taille des pores est inférieure ou égale au poids moléculaire du FVII permet de concentrer le produit sans perte notable. Par exemple, une membrane de taille de pores à 50 kDa permet de concentrer sans perte du FVII dont le poids moléculaire est de 50 kDa.

La dialyse est destinée à conditionner la phase aqueuse non lipidique comprenant le FVII pour des étapes ultérieures de purification, notamment par chromatographie. Elle permet également d'éliminer les composants de faible taille moléculaire, comme les lactoses, les sels, les peptides, les protéoses peptones et tout agent pouvant nuire à la conservation du produit. A cet effet, on utilise préférentiellement une membrane ayant un seuil de rétention de 50 kDa, de sorte que le FVII n'est pas filtré à travers la membrane.

De préférence, le tampon de dialyse est une solution de phosphate de sodium 0,025 M-0,050 M, pH 7,5-8,5.

La phase aqueuse non lipidique obtenue après l'étape b) ou, le cas échéant, telle qu'obtenue après les étapes de filtration et/ou de concentration/dialyse, peut être congelée et stockée à une température de -30°C dans l'attente de la mise en oeuvre d'étapes ultérieures de leur purification.

La phase aqueuse non lipidique obtenue selon l'étape b) est ensuite soumise à une étape c) de chromatographie d'affinité, en utilisant des dispositifs classiques de chromatographie, avantageusement mise en oeuvre sur une colonne chromatographique dont le support est un gel d'hydroxyapatite (Ca₁₀(PO₄)₆(OH)₂) ou un gel de fluoroapatite (Ca₁₀(PO₄)₆F₂). Ainsi, le FVII de la phase aqueuse est retenu par le support, la majeure partie des protéines lactiques non retenue étant éliminée.

On utilise de préférence une colonne chromatographique qui est équilibrée en tampon aqueux A à base de phosphate de sodium 0,025 M-0,035 M, pH 7,5-8,5. La phase aqueuse enrichie en FVII est injectée sur la colonne, ce qui permet la rétention du FVII. La fraction non retenue est éliminée par percolation du tampon A jusqu'au retour à la ligne de base (RLB), ce qui assure une bonne élimination des composés indésirables, telles que les protéines lactiques. La détection des composés est assurée par mesure de l'absorbance à λ= 280 nm.

L'élution du FVII selon l'étape c) est effectuée par un tampon à base d'un sel de phosphate, tel que le phosphate de sodium ou de potassium ou leur mélange, à une concentration prédéterminée, de préférence représentant un tampon B à base de phosphate de sodium 0,25 M-0,35 M, pH 7,5-8,5. La fraction éluée est collectée jusqu'au RLB. La détection est assurée par mesure de l'absorbance à λ= 280 nm.

Grâce à cette étape, plus de 90% du total des protéines lactiques sont éliminés et plus de 90% des du FVII sont récupérées. La pureté de cette fraction éluée de FVII est d'environ 5% à cette étape.

La pureté est définie comme étant le rapport massique entre le FVII et les protéines totales présentes dans l'échantillon, la fraction ou l'éluat considéré.

Avantageusement, l'activité spécifique du FVII est accrû d'un facteur 10 à 25 du fait de l'affinité du FVII au support chromatographique.

L'éluat de FVII obtenu à l'issue de l'étape c) est ensuite avantageusement soumis à une filtration tangentielle.

La membrane de filtration tangentielle est choisie par l'homme du métier en fonction des caractéristiques du FVII. De façon générale, un seuil de coupure dont la taille des pores est deux fois supérieure au poids moléculaire du FVII permet de le filtrer. Par conséquent, une membrane de tailles de pores de 100 kDa permet de filtrer le FVII avec un bon rendement.

Le but de cette étape de filtration est de réduire la charge notamment en protéines de poids moléculaire supérieur à celui du FVII et, en particulier, d'éliminer les formes atypiques du FVII (par exemple du FVII sous forme polymérisée), ainsi que des protéases susceptibles, à terme, de le dégrader. A cette fin, on utilise très avantageusement une membrane de seuil de rétention de composés de 100 kDa.

Avantageusement, l'éluat de FVII filtré obtenu est ensuite concentré et dialysé par ultrafitration sur des membranes de seuil de rétention de 50 kDa. Le tampon de dialyse est de préférence une solution de chlorure de sodium 0,15 M-0,20 M.

L'éluat de FVII obtenu à l'issue de l'étape c), éventuellement filtré, concentré et dialysé, est ensuite soumis à deux ou trois étapes de chromatographie (étape d)) sur des colonnes échangeuses d'anions de type base faible utilisant des tampons appropriés pour des élutions successives du facteur VII retenu sur lesdites colonnes. Ces étapes permettent une purification supplémentaire du FVII notamment au regard de protéines lactiques. Elles sont mises en oeuvre avec des dispositifs classiques de chromatographie.

La première étape chromatographique d'échange d'anions est avantageusement mise en oeuvre grâce à un support chromatographique de type gel Q-Sepharose® FF sur lequel le facteur VII est retenu, selon les conditions opératoires préférentielles qui suivent. Ce support est lavé par un tampon Tris 0,05 M, pH 7,0-7,5. La fraction non retenue est éliminée par le tampon de lavage jusqu'au retour à la ligne de base. L'élution du FVII est effectuée par un tampon aqueux à base de Tris 0,05 M et de chlorure de calcium 0,020 M-0,05 M, de préférence 0,05 M, pH 7,0-8,0, afin d'obtenir un premier éluat de facteur VII. La détection des composés est assurée par mesure de l'absorbance à λ= 280 nm.

Par cette étape, il est récupéré au moins 70% du FVII et celle-ci permet une élimination d'environ 80% de protéines accompagnantes.

L'éluat de FVII peut ensuite être soumis à une étape de dialyse, comme décrit précédemment, dont le tampon est une solution de chlorure de sodium 0,15-0,20 M.

La deuxième étape chromatographique est avantageusement mise en oeuvre grâce un support chromatographique de type gel Q-Sepharose® FF sur -lequel est injecté le premier éluat de FVII, obtenu par la première étape chromatographique sur échangeur d'anions, selon les conditions opératoires préférentielles qui suivent. Le support est lavé par un tampon Tris 0,05 M, pH 7,0-7,5. La fraction non retenue est éliminée par le tampon de lavage jusqu'au retour à la ligne de base. La détection est assurée par mesure de l'absorbance à λ= 280 nm.

L'élution du FVII, retenu sur le support, est effectuée par un tampon aqueux à base de Tris 0,05 M et de chlorure de calcium 0, 005 M, pH 7,0-8,0, pour l'obtention d'un deuxième éluat de facteur VII de haute pureté, soit d'une purété supérieure à 90%. La détection est assurée par mesure de l'absorbance à λ= 280 nm.

La deuxième étape chromatographique est destinée à limiter une éventuelle dégradation protéolytique de la protéine.

A ce stade, la pureté de l'éluat est d'environ 90%, et plus de 95% de protéines accompagnantes restantes sont éliminées. Il peut ensuite être soumis à une étape de dialyse, comme décrit précédemment, dont le tampon est une solution de chlorure de sodium 0,15-0,20 M.

Selon une mise en oeuvre très préférée de l'invention, le procédé comprend trois étapes de chromatographie sur des colonnes échangeuses d'anions de type base faible utilisant des tampons appropriés pour des élutions successives du facteur VII à partir desdites colonnes. Il est ainsi mis en oeuvre, après les deux étapes chromatographiques d'échange d'anions ci-dessus, une troisième étape chromatographique d'échange d'anions. Cette étape permet la formulation de la composition enrichie en la protéine, de manière à la rendre adaptée à une utilisation médicale.

La troisième étape chromatographique est avantageusement mise en oeuvre grâce à un support chromatographique de type gel Q-Sepharose® FF sur lequel est injecté le deuxième éluat de FVII, obtenu par la deuxième étape chromatographique sur échangeur d'anions, selon les conditions opératoires préférentielles qui suivent.

Le deuxième éluat obtenu par la deuxième étape chromatographique d'échange d'anions est injecté, de préférence après dilution par 1 à 5 volumes d'eau purifiée pour injection (PPI), sur une colonne remplie de support de type gel Q-Sepharose® FF sur lequel le facteur VII est retenu. Ce support est lavé par un tampon Tris 0,05 M, pH 7,0-7,5. La fraction non retenue est éliminée par le tampon de lavage jusqu'au retour à la ligne de base. La détection est assurée par mesure de l'absorbance à λ= 280 nm.

L'élution du FVII, retenu sur le support, est effectuée par un tampon aqueux à base de Tris 0,02 M et de chlorure de sodium 0,20-0,30 M, de préférence 0,28 M, pH 6,5-7,5. La détection est assurée par mesure de l'absorbance à λ= 280 nm.

La composition de FVII trangénique ainsi obtenue a une pureté supérieure à 95%. Très avantageusement, la composition se présente sous la forme d'un concentré de FVII.

La mise en oeuvre du procédé conduit à un rendement cumulé de 20 à 25%, ce qui permet de purifier au moins 20 mg de FVII/litre de lait traité.

Par conséquent, les trois étapes de chromatographies sur gel échangeur d'anions permettent de purifier encore davantage le FVII. De plus, elles permettent la concentration et la formulation de la composition de FVII.

L'activation du FVII intervient au cours du procédé, selon toute vraisemblance au cours de la première étape de chromatographie d'échange d'anions.

Après la récupération du dernier éluat, celui-ci peut être soumis à une filtration sur des filtres de 0,22 µm, de répartition dans des flacons et congelés à -30°C et conservés à cette température.

Le procédé de l'invention peut comprendre également au moins l'une des étapes suivantes de formulation, inactivation virale et stérilisation. De façon générale, le procédé peut comprendre, avant l'étape de chromatographie d'affinité, une étape de traitement anti-viral qui est avantageusement effectuée par solvant/détergent, en particulier en présence d'un mélange de Tween® 80 (1% p/v) et de TnBP (tri-n-butylphosphate) (0,3% v/v,), ce qui permet d'inactiver les virus enveloppés. En outre, l'éluat de FVII issu de la deuxième étape chromatographique sur échangeur d'anions est de préférence soumis à une étape de nanofiltration pour une élimination efficace des virus, en particulier des virus non enveloppés, tels que le parvovirus B19. Il est possible d'utiliser des filtres ASAHI PLANOVA™15 permettant la rétention des virus ayant une taille supérieure à 15 nm.

Un autre objet de l'invention est la composition de FVII de l'invention pour une utilisation comme médicament.

Un autre objet de l'invention est l'utilisation de la composition de FVII selon l'invention pour la préparation d'un médicament destiné au traitement des patients atteints d'hémophilie.

Un autre objet de l'invention est l'utilisation de la composition de FVII selon l'invention pour la préparation d'un médicament destiné au traitement des traumatismes hémorragiques multiples.

Un autre objet de l'invention est l'utilisation de la composition de FVII selon l'invention pour la préparation d'un médicament destiné au traitement des saignements massifs non contrôlables par une hémostase chirurgicale.

Un autre objet de l'invention est l'utilisation de la composition de FVII pour la préparation d'un médicament destiné au traitement des hémorragies dues à un surdosage en anticoagulants.

Un autre objet de l'invention est une composition pharmaceutique comprenant le facteur VII selon l'invention et un excipient et/ou un véhicule pharmaceutiquement acceptables.

L'excipient peut être toute solution, telle qu'une solution saline, physiologique, isotonique ou tamponnée, ainsi que toute suspension, gel ou poudre, compatible avec un usage pharmaceutique et connu de l'homme du métier. Les compositions selon l'invention peuvent en outre contenir un ou plusieurs agents ou véhicules choisis parmi les dispersants, solubilisants, stabilisants, surfactants et conservateurs. D'autre part, les compositions selon l'invention peuvent comprendre d'autres agents ou principes actifs.

Par ailleurs, les compositions peuvent être administrées de différentes manières et sous différentes formes. L'administration peut être réalisée par toute voie classique pour ce type d'approche thérapeutique, comme notamment par voie systémique, en particulier par injection intraveineuse, intradermique, intra-tumorale, sous-cutanée, intra-péritonéale, intramusculaire ou intra-artérielle. On peut citer par exemple l'injection intra-tumorale ou l'injection dans une zone proche de la tumeur ou irriguant la tumeur.

Les doses peuvent varier en fonction du nombre d'administrations, de l'association à d'autres principes actifs, du stade d'évolution de la pathologie, etc.

D'autres aspects et avantages de l'invention seront décrits dans les exemples qui suivent, qui doivent être considérés comme illustratifs et ne limitent pas l'étendue de l'invention.

### Abbréviations pour les exemples

FVII-tg = FVIIa-tg: FVII transgénique activé selon l'invention
FVII-rec = FVIIa-rec : FVII recombinant activé disponible dans le commerce
FVII-pd = FVIIa-pd : FVII d'origine plasmatique activé, c'est-à-dire purifié à partir de plasma humain.
MALDI-TOF : Matrix Assisted Laser Desorption Ionisation - Time of Flight
HPCE-LIF : High Performance Capillary Electrophoresis-Laser Induced Fluorescence (Electrophorèse capillaire haute performance-fluorescence induite par laser) ESI-MS : Spectrométrie de masse-Ionisation « Electrospray ».
LC-ESIMS : Chromatographie liquide- Spectrométrie de masse-Ionisation « Electrospray »
NP-HPLC : Chromatographie Liquide Haute Performance en phase normale
PNGase F : Peptide : N-glycosidase F
LC-MS : Chromatographie liquide- Spectrométrie de masse

### Description des figures

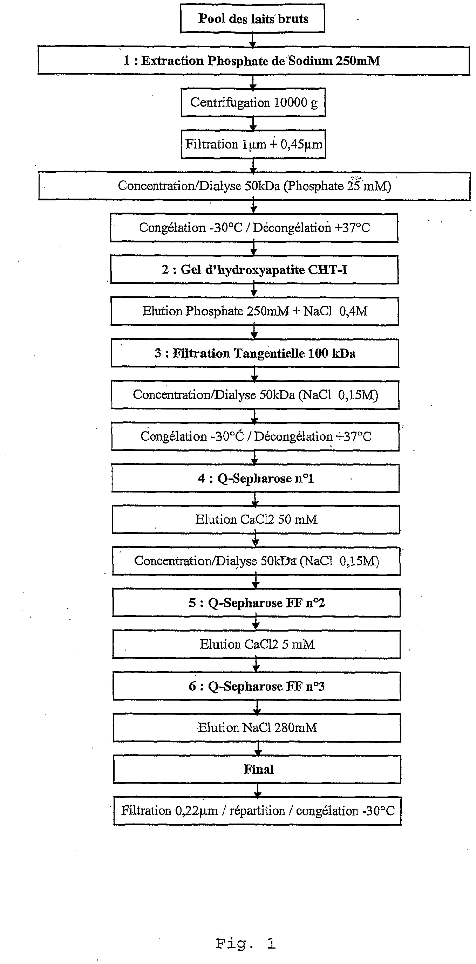

Figure 1 : schéma d'un exemple du procédé d'extraction et de purification/activation du FVII transgénique selon l'invention produit dans le lait de lapine.
Figure 2 : Spectres de masse ESI déconvolués des peptides porteurs des sites de N-glycosylation.
Figure 3 : Electrophérogrammes HPCE-LIF après déglycosylation du FVII par la PNGase F ;
   Légende : les deux électrophérogrammes du milieu : FVII-tg ; (◁) fucose, (■) GlcNAc, (●) mannose, (●) galactose, (▲) acide sialique. Electrophérogramme du haut : FVII-pd Electrophérogrammes du milieu (2) : FVII-tg Electrophérogramme du bas : FVII-rec
Figure 4 : Caractérisation du FVII-tg (chromatogramme du milieu) par NP-HPLC, du FVII-pd (chromatogramme du haut) et du FVII-rec (chromatogramme du bas).
   Légende : idem figure 3.
Figure 5 : Identification des formes glycanniques majoritaires du FVII-tg par MALDI-TOFMS. Légende des résidus de sucre : idem figure 3
Figure 6 : Analyse en HPCE-LIF des oligosaccharides de la Thyroglobuline. Légende des résidus de sucre : idem figure 3
Figure 7 : Analyse en HPCE-LIF des oligosaccharides du FVII transgénique. Légende : idem figure 3
Figure 8 : Quantification des structures Galα1,3Gal.
Figure 9 : Exemple d'un mécanisme d'extraction du FVII
Figure 10 : Spectres de masse ESI déconvolués des peptides porteurs des sites de O-glycosylation. Ces peptides ont été obtenus après digestion à la trypsine et analyse par LC-ESIMS. Fuc : fucose, Glc : glucose, Xyl : xylose.
Figure 11. Spectres de masse ESI déconvolués des peptides porteurs des sites de γ-carboxylation. Ces peptides ont été obtenus après digestion à l'Asp-N et analyse par LC-ESIMS. Les résultats obtenus pour les différents lots de FVII-tg sont très similaires. Les masses des différents peptides sont des masses monoisotopiques. Gla : acide γ-carboxylique.

### Exemples

### Exemple . 1 : Production de lapines transgéniques produisant une protéine de FVII humain dans leur lait

On prépare tout d'abord un plasmide p1 en introduisant la séquence Bam H1-Hind III (fragment 6,3 Kb) du gène WAP (décrite dans le document Devinoy et al, Nucleic Acids Research, vol. 16, no. 16, 25 août 1988, p. 8180) dans le polylinker du vecteur p-poly III-I (décrit dans le document Lathe et al, Gene (1987) 57, 193-201), entre les séquences Bam H1 et Hind III.

Au cours de ce clonage, le site Bam H1 a été supprimé et remplacé par le site Cla I qui figure dans le vecteur p1. Le vecteur p1 est donc un plasmide capable de recevoir un gène étranger placé sous la dépendance du promoteur WAP 6,3 Kb. L'introduction du gène étranger peut se faire par exemple dans le site Sal I du poly linker. Les inserts contenant la totalité du promoteur et des gènes étrangers peuvent être isolés du plasmide après une coupure aux deux sites Not 1 qui sont aux extrémités du poly linker du plasmide p-poly III-I.

Le plasmide p2, obtenu à partir du plasmide p1, contient le promoteur du gène de la WAP de lapin (6,3 Kb) et le gène du FVII humain. Le fragment utilisé pour obtenir les lapines transgéniques est compris entre les deux sites Not1.

Un site Hind III a été introduit dans la séquence de tête du gène (leader) par mutagénèse dirigée pour servir de site de clonage.

Les lapines transgéniques ont été obtenues par la technique classique de microinjection (Brinster et al, Proc. Natl. Acad. Sci. USA (1985) 82, 4438-4442). 1-2 pl contenant 500 copies du gène ont été injectés dans le pronucleus mâle d'embryons de souris. Les constructions ont été réalisées dans le vecteur p-poly III-I (Lathe et al, Gene (1987) 57, 193-201). Les fragments Not 1 - Not 1 de ce vecteur contenant les gènes recombinés ont été microinjectés. Les embryons ont ensuite été transférés dans l'oviducte de femelles adoptives hormonalement préparées. Environ 10% des embryons manipulés ont donné naissance à des lapereaux et 2-5% des embryons manipulés à des lapereaux transgéniques. La présence des transgènes a été révélée par la technique de transfert de Southern à partir de l'ADN extrait des queues des lapins. Les concentrations de FVII dans le sang et dans le lait des animaux ont été évaluées à l'aide de tests radioimmunologiques spécifiques.

L'activité biologique du FVII a été évaluée en ajoutant du lait au milieu de culture de cellules ou d'explants mammaires de lapin.

La technique utilisée pour l'obtention de lapines transgéniques produisant dans leur lait le FVII de l'invention est décrite plus en détails dans le document EP 0 527 063.

### Exemple 2 : Extraction et purification du FVII obtenu

### a) Extraction du FVII

On considère 500 ml de lait brut non écrémé qui sont dilués par 9 volumes de tampon phosphate de sodium 0,25 M, pH 8,2. Après 30 minutes d'agitation à température ambiante, la phase aqueuse enrichie en FVII est centrifugée à 10 000g durant 1 heure à 15°C (centrifugeuse Sorvall Evolution RC - 6700 tours/min - rotor SLC-6000). 6 pots d'environ 835 ml sont nécessaires.

Après centrifugation, trois phases sont présentes : une phase lipidique en surface (crème), une phase aqueuse non lipidique claire enrichie en FVII (phase majoritaire) et une phase blanche solide en culot (précipités de caséines non solubles et de composés de calcium).

La phase aqueuse non lipidique comprenant le FVII est collectée à la pompe péristaltique jusqu'à la phase crémeuse. La phase crémeuse est collectée à part. La phase solide (précipité) est éliminée.

La phase aqueuse non lipidique, comprenant toutefois encore de très faibles quantités de lipides, est filtrée sur une séquence de filtres (Pall SLK7002U010ZP - préfiltre en fibres de verre de taille de pores de 1 µm - puis Pall SLK7002NXP - Nylon 66 de taille de pores de 0,45 µm). En fin de filtration, la phase lipidique est passée sur cette séquence de filtration qui retient complètement les globules lipidiques du lait, et le filtrat est clair.

La phase aqueuse non lipidique filtrée est ensuite dialysée sur membrane d'ultrafiltration (Millipore Biomax 50 kDa - 0,1 m²) pour la rendre compatible avec la phase de chromatographie. Le FVII de poids moléculaire d'environ 50 kDa ne filtre pas à travers la membrane, contrairement aux sels, sucres et peptides du lait. Dans un premier temps, la solution (environ 5 000 ml) est concentrée à 500 ml, puis une dialyse par ultrafiltration en maintenant le volume constant permet d'éliminer les électrolytes et de conditionner la matière biologique pour l'étape de chromatographie. Le tampon de dialyse est un tampon phosphate de sodium 0,025M, pH 8,2.

On peut assimiler cette phase aqueuse non lipidique comprenant le FVII à du lactosérum enrichi en FVII-tg. Cette préparation est stockée à -30°C avant la poursuite du procédé.

Le rendement global de récupération du FVII par cette étape est très satisfaisant : 90% (91% extraction au phosphate + 99% Dialyse/concentration).

La phase aqueuse non lipidique comprenant le FVII à l'issue de cette étape est parfaitement limpide et est compatible avec les étapes chromatographiques qui font suite.

Environ 93 000 UI de FVII-tg sont extraites à ce stade. La pureté en FVII de cette préparation est de l'ordre de 0,2%.

### b) Purification du FVII

### 1. Chromatographie sur gel d'hydroxyapatite

Une colonne Amicon 90 (9 cm de diamètre - 64 cm² de section) est remplie avec du gel BioRad Ceramic Hydroxyapatite type I (CHT-I).

Le gel est équilibré en tampon aqueux A constitué d'un mélange de phosphate de sodium 0,025 M et de chlorure de sodium 0,04 M, pH 8,0. La totalité de la préparation conservée à -30°C est décongelée au bain-marie à 37°C jusqu'à dissolution complète du glaçon puis est injectée sur le gel (débit linéaire 100 cm/h, soit 105 ml/min). La fraction non-retenue est éliminée par passage d'un tampon constitué de phosphate de sodium 0,025 M et de chlorure de sodium 0,04 M, pH 8,2, jusqu'au retour à la ligne de base (RLB) .

L'élution de la fraction contenant le FVII-tg se fait par le tampon B constitué de phosphate de sodium 0,25 M et de chlorure de sodium 0,4 M, pH 8,0. La fraction éluée est collectée jusqu'au retour à la ligne de base. La détection des composés est assurée par mesure de l'absorbance à λ= 280 nm.

Cette chromatographie permet de récupérer plus de 90% du FVII-tg, tout en éliminant plus de 95% des protéines lactiques. L'activité spécifique (A.S.) est multipliée par 25. Environ 85 000 UI de FVII-tg de pureté de 4% sont disponibles à ce stade.

### 2. Filtration tangentielle 100 kDa et concentration/dialyse 50 kDa

La totalité de l'éluat de l'étape précédente est filtrée en mode tangentiel sur une membrane d'ultrafiltration 100 kDa (Pall OMEGA SC 100K - 0,1 m²). Le FVII est filtré à travers la membrane 100 kDa, alors que les protéines de poids moléculaire supérieur à 100 kDa ne sont pas filtrables.

La fraction filtrée est ensuite concentrée à environ 500 ml, puis dialysée sur l'ultrafiltre 50 kDa déjà décrit plus haut. Le tampon de dialyse est du chlorure de sodium 0,15 M.

A ce stade du procédé, le produit est stocké à - 30°C avant passage en chromatographie d'échange d'ions.

Cette étape a permis de réduire la charge en protéines de poids moléculaire supérieur à 100 kDa et en particulier des pro-enzymes. Le traitement membranaire 100 kDa permet de retenir environ 50% des protéines dont les protéines de haut poids moléculaire, tout en filtrant 95% du FVII-tg, soit 82 000 UI de FVII-tg.

Ce traitement permet de réduire les risques d'hydrolyse protéolytique lors des étapes avales.

### 3. Chromatographies sur gel Q-Sepharose® FF

Ces trois chromatographies successives sur gel échangeur d'ions Q-Sepharose® Fast Flow (QSFF) sont réalisées pour purifier le principe actif, permettre l'activation du FVII en FVII activé (FVIIa) et finalement concentrer et formuler la composition en FVII. La détection des composés est assurée par mesure de l'absorbance à λ= 280 nm.

### 3.1 Etape Q-Sepharose® FF 1 - élution « High Calcium »

Une colonne de 2,6 cm de diamètre (5,3 cm² de section) est remplie de 100 ml de gel Q-Sepharose® FF (GE Healthcare).

Le gel est équilibré en tampon Tris 0,05 M, pH 7,5.

La totalité de la fraction conservée à -30°C est décongelée au bain-marie à 37°C jusqu'à dissolution complète du glaçon. La fraction est diluée au ½ [v/v] avec le tampon d'équilibrage avant injection sur le gel (débit 13 ml/min, soit débit linéaire de 150 cm/h), puis la fraction non retenue est éliminée par passage du tampon jusqu'au RLB.

Une première fraction protéique à faible teneur en FVII est éluée à 9 ml/min (soit 100 cm/h) par un tampon de Tris 0,05 M et de chlorure de sodium 0,15 M, pH 7,5, et est ensuite éliminée.

Une deuxième fraction protéique riche en FVII est éluée à 9 ml/min (soit 100 cm/h) par un tampon de Tris 0,05 M, de chlorure de sodium 0,05 M et de chlorure de calcium 0,05 M, pH 7,5.

Cette deuxième fraction est dialysée sur l'ultrafiltre 50 kDa déjà décrit plus haut. Le tampon de dialyse est du chlorure de sodium 0,15 M. Cette fraction est conservée à +4°C durant une nuit avant le 2^{ème} passage en chromatographie d'échange d'anions.

Cette étape permet de récupérer 73% du FVII (soit 60000 UI de FVII-tg), tout en éliminant 80% des protéines accompagnantes. Elle permet également l'activation du FVII en FVIIa.

### 3.2 Etape Q-Sepharose® FF 2 - élution « Low Calcium »

Une colonne de 2,5 cm de diamètre (4,9 cm² de section) est remplie de 30 ml de gel Q-Sepharose® FF (GE Healthcare).

Le gel est équilibré en tampon Tris 0,05 M, pH 7,5.

La fraction éluée précédente (deuxième fraction), conservée à +4°C, est diluée avant injection sur le gel (débit 9 ml/min, soit un débit linéaire de 100 cm/h).

Après injection, le gel est lavé par le tampon d'équilibrage pour l'élimination de la fraction non retenue.

Une fraction contenant du FVII de très haute pureté est éluée à 4,5 ml/min (soit 50 cm/h) en tampon de Tris 0,05 M, de chlorure de sodium 0,05 M et de chlorure de calcium 0,005 M, pH 7,5.

Environ 23 000 UI de FVII-tg ont été purifiées, soit 12 mg de FVII-tg.

Cette étape permet d'éliminer plus de 95% des protéines accompagnantes (protéines du lait de lapine).

Cet éluat, de pureté supérieure à 90%, présente des caractéristiques structurales et fonctionnelles proches des molécules naturelles du FVII humain. Il est concentré et formulé par le troisième passage en chromatographie d'échanges d'ions.

### 3.3 Etape Q-Sepharose® FF 3 - élution « Sodium »

Une colonne de 2,5 cm de diamètre (4,9 cm² de section) est remplie de 10 ml de gel Q-Sepharose® FF (GE Healthcare).

Le gel est équilibré en tampon Tris 0,05 M, pH 7,5.

La fraction éluée purifiée de l'étape précédente est diluée cinq fois avec de l'eau purifiée pour injection (PPI) avant injection sur le gel (débit 4,5 ml/min, soit débit linéaire de 50 cm/h).

Après injection, le gel est lavé par le tampon d'équilibrage pour l'élimination de la fraction non retenue.

Le FVII-tg est ensuite élué à un débit de 3 ml/min (soit 36 cm/h) par le tampon de Tris 0,02 M et de chlorure de sodium 0,28 M, pH 7,0.

Une composition de FVII-tg sous forme de concentré a été préparée dont la pureté est supérieure à 95%. Le produit est compatible avec une injection par voie intraveineuse. Le procédé a un rendement cumulé de 22%, ce qui permet de purifier au moins 20 mg de FVII par litre de lait mis en oeuvre.

Le FVII-tg de la composition est ensuite soumis à différentes analyses structurales, telles que développées dans les exemples qui suivent.

### Exemple 3 : Etude de la séquence primaire

### 1. Carte peptidique

Les chromatogrammes LC-MS et détection UV après digestion à la trypsine et à l'Asp-N des échantillons FVII-pd, FVII-rec et FVII-tg, selon des mises en oeuvre connues de l'homme du métier, ont été obtenus (données non montrées). L'analyse des cartes peptidiques obtenue après digestion à la trypsine montrent qu'elles sont semblables pour des temps de rétention inférieurs à 65 min. Au delà, des espèces correspondant à des fragments peptidiques de la chaîne lourde sont observés dans le FVII-rec et le FVII-tg et pas dans le FVII-pd. Ces peptides qui ont des masses élevées (4500 Da à 8000 Da) correspondent à des clivages manqués. Ces peptides ont été observés pour d'autres lots de FVII-pd, leur présence ne peut donc être reliée à une susceptibilité à la trypsine différente.

Les cartes peptidiques obtenues après digestion à l'Asp-N sont très similaires d'un FVII à l'autre et ce, sur toute la gamme de temps de rétention. Les différences d'intensité observées ne sont dues qu'à des variations de quantités injectées et ne sont pas à relier avec des différences structurales.

### 2. Couverture de séquence

Les recouvrements de séquence sont calculés à partir des peptides identifiés par MS et/ou MS/MS. Le Tableau 1 regroupe les résultats obtenus pour les différents échantillons. En considérant les digestions par la trypsine et l'Asp-N, plus de 95% de la séquence primaire a pu être vérifiée, les résultats obtenus sont en accord avec ceux décrits dans la littérature (Thim L. et al, Biochemistry, 1988, 27, 7785-7793).

**Tableau 1. Recouvrements de séquence obtenus par LC-ESIMS(/MS) pour les différents échantillons analysés.**

| | Recouvrement trypsine (%) | Recouvrement Asp-N (%) | Recouvrement global (%) |
|---|---|---|---|
| FVII-pd | 79,3 | 97,3 | 97,3 |
| FVII-rec | 97,5 | 95,8 | 100,0 |
| FVII-tg | - | 97,0 | 97,0 |
| FVII-tg | 96,6 | 91,1 | 100,0 |
| FVII-tg | 95,3 | 91,1 | 99,0 |

Afin de confirmer l'identité des peptides, les fractions HPLC ont été analysées par MALDI et par séquençage d'Edman.

L'ensemble des fractions HPLC analysées par séquençage d'Edman permet d'obtenir un recouvrement de séquence de 80%. Les vérifications de séquence, réalisées sur les FVII-tg et FVII-rec en LC-MS, donnent des couvertures de séquence primaire > 95%.

### Exemple 4 : Caractérisation des sites de glycosylation et des glycopeptides par ESI-MS

Les sites de N-glycosylation du FVII-tg ont été identifiés par LC-ESIMS(/MS), confirmés par MALDI-TOFMS, et la microhétérogénéité a été déterminée par LC-ESIMS.

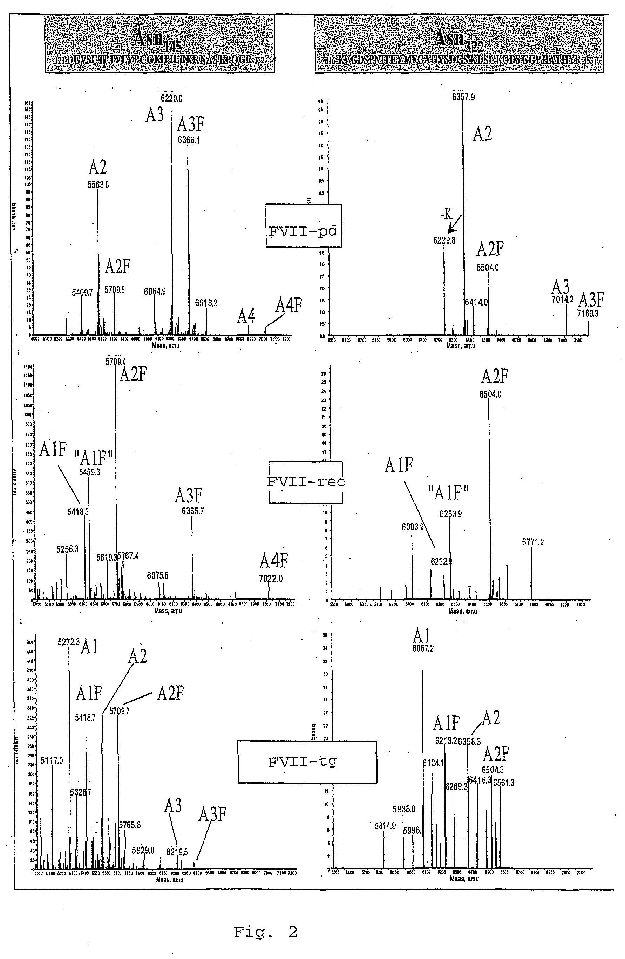

La figure 2 représente les spectres ESI déconvolués des glycopeptides contenant les deux résidus Asn glycosylés. La localisation des sites de glycosylation a été confirmée par MALDI-TOF(/TOF) ainsi que par séquençage d'Edman.

L'analyse des spectres de masse des glycopeptides [D₁₂₃-R_{152]} et [K_{31.6}-R₃₅₃] du FVII plasmatique (FVII-pd), possédant respectivement les sites de N-glycosylation Asn₁₄₅ et Asn₃₂₂, révèle la présence de structures biantennées, bisialylées, non fucosylées (A2) (masse observée du glycopeptide : 5563.8 Da) et fucosylées (A2F) (masse obs : 5709.8 Da). On note également la présence d'oligosaccharides triantennés, trisialylés non fucosylés (A3) (masse obs. 6220.0 Da) et fucosylés (A3F) (masse obs. 6366.1 Da). Pour le FVII transgénique (FVII-tg), les oligosaccharides situés sur l'Asn₁₄₅ sont majoritairement de type biantennés, monosialylés non fucosylés ou fucosylés (A1, A1F) et A2, A2F. Les formes triantennées sont peu représentées.

Concernant les glycoformes majoritaires de l'Asn₃₂₂, on observe les mêmes structures glycanniques dans des proportions différentes. La figure 2 révèle la présence de formes moins matures (moins antennées et sialylées) que sur l'Asn₁₄₅. Par exemple, les structures trianténnées sont moins représentées sur Asn₃₂₂ que sur Asn₁₄₅ pour le produit plasmatique et absentes sur le FVII-tg Il faut noter également que les Asn 145 et 322 sont glycosylées à 100%. Bien que semi-quantitatifs, ces résultats sont en accord avec les données quantitatives obtenus par HPCE-LIF et NP-HPLC.

### Exemple 5 : Quantification des N-glycannes par HPCE-LIF

L'identification et la quantification des oligosaccharides N-liés sont réalisées par HPCE-LIF après déglycosylation par la PNGase F. Les échantillons de FVII sont traités par des exoglycosidases (sialidase (ratio ENZYME/SUBSTRAT 1 mUI/10 µg), galactosidase, hexnacase (kit Prozyme), fucosidase (ratio E/S : 1 mUI/10 µg) de façon à s'assurer de l'identification et de la quantification de chaque structure isolée. Les glycannes obtenus sont marqués par un fluorochrome et séparés en fonction de leur masse et de leur charge. Deux standards (homopolyméres de glucose, oligosaccharidiques) permettent d'identifier les structures. La quantification se fait par l'intégration de chaque pic ramenée, en pourcentage, à l'ensemble des oligosacharides quantifiés.

On utilise un appareillage d'électrophorèse capillaire ProteomeLab PA800 (Beckman Coulter) dont le capillaire est « coaté » N-CHO (Beckman-Coulter) de 50 cm x 50 µm de diamètre interne. Il est utilisé un tampon de séparation «gel buffer-N » (Beckman-Coulter). La migration est effectuée par l'application d'une tension de 25 kV, pendant 20 min, à 20°C. La détection a lieu par laser à λ_{excitation} 488nm et λₑₘᵢₛₛᵢₒₙ 520 nm.

Le taux de fucosylation est calculé, après déglycosylation simultanée par les sialidase, galactosidase et hexnacase, par le rapport entre les surfaces des pics correspondant au "core" et "core" fucosylé.

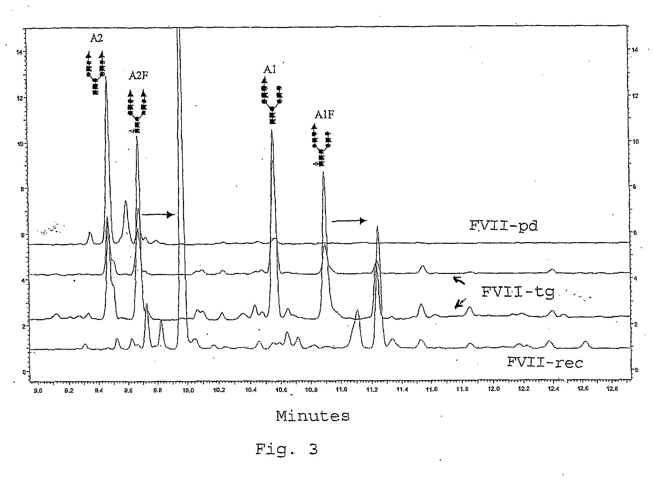

Les glycannes du FVIIa-pd sont majoritairement de types biantennés bisialylés (A2), et biantennés bisialylés fucosylés (A2F). Les profils glycaniques (cf figure 3) des FVII-tg révèlent la présence de structures biantennées, monosialylées, fucosylées ou non (A1F, A1), et biantennées, bisialylées, fucosylées ou non (A2, A2F). Le profil du FVII-rec révèle des temps de migration atypique pour les différentes structures (A1F, A2F) observées.

**Tableau 2. Récapitulatif du pourcentage des structures sialylées issues des échantillons natifs (non désialylés) et celles issues des échantillons désialylés des différents lots de FVII : FVII-tg et FVII-pd**

| Pourcentages | FVII-pd | FVII-tg Lot A | FVII-tg Lot B |
|---|---|---|---|
| *Natif* | | | |
| A2 | 41.9 | 19.3 | 13.9 |
| A2F | 8.9 | 14.8 | 21.5 |
| A1 | 2.6 | 38.4 | 25.2 |
| A1F | - | 11.7 | 22.2 |
| Total A2+A2F | 50.8 | 34.1 | 35.4 |
| Total A1+A1F | 2.6 | 50.1 | 47.4 |

| *Désialylé* | | | |
|---|---|---|---|
| G2 | 44.5 | 57.5 | 37.2 |
| G2F | 8.9 | 18 | 38.1 |
| G3 | 25 | 4.6 | 3.0 |
| G3F | 5.1 | 1.3 | 3.0 |
| Autres espèces non fucosylées | 12.6 | 2.9 | 3.0 |
| Autres espèces fucosylées | 2.2 | 3.6 | 7.5 * |
| G2FB | - | - | - |

| | | | |
|---|---|---|---|
| *espèce bifucosylée G2F, G2 : structures biantennées, bigalactosylées, fucosylées ou non fucosylées G2FB : structures biantennées, bigalactosylées, fucosylées et bisectées | | | |

L'analyse quantitative des différentes structures glycanniques (Tableau 2) montre que pour le FVII-pd, la prédominance de structures sialylées avec environ 51% de glycannes bisialylées (A2 et A2F) et 30% de formes triantennées, sialylées non fucosylées ou fucosylées (G3 et G3F). Le FVII-tg (lots A et B) est moins sialylé que le FVII-pd avec 35% de formes biantennées bisialylées et seulement 6% de formes triantennés sialylées. Les formes majoritaires sont monosialylées avec 50% de structures A1 et A1F. Le FVII-rec est également moins sialylé que le FVII-pd avec 45% de structures A2F et seulement 6% de glycannes trianténnés sialylées (résultats non montrés).

On note pour le FVII-rec le faible taux de structures non fucosylées.

**Tableau 3. Taux de fucosylation des différents FVII**

| | FVII-pd | FVII-tg Lot A | FVII-tg Lot B | FVII-rec |
|---|---|---|---|---|
| Taux de fucosylation(%) | 16.2 | 23.6 | 41.8 | 100 |

L'analyse quantitative montre un faible taux de fucosylation du FVII-pd (16%), un taux de fucosylation de 24 à 42% pour le FVII-tg et une fucosylation de 100% pour le FVII-rec.

### Exemple 6 : Quantification des N-glycannes par NP-HPLC

Les analyses qualitative et quantitative de la N-glycosylation des FVII-pd et FVII-tg a été étudiée par NP-HPLC. Après dessalage et séchage de la protéine, celle-ci est dénaturée et réduite selon des mises en oeuvre connues de l'homme du métier. Les glycannes sont ensuite libérés par voie enzymatique (endoglycosidase PNGase F), et purifiés par précipitation à l'éthanol. Les glycannes ainsi obtenus sont marqués à l'aide d'un fluorophore, le 2-aminobenzamide (2-AB). Les glycannes marqués sont séparés en fonction de leur hydrophylie par chromatographie HPLC en phase normale sur colonne Amide-80, 4,6 x 250 mm (Tosohaas) thermostatée à 30°C. Avant injection de l'échantillon, la colonne est équilibrée en tampon à 80 % acétonitrile. Les oligosaccharides sont élués par un gradient croissant de formiate d'ammonium 50 mM, pH 4.45, pendant des temps supérieurs ou égaux à 140 minutes. La détection est réalisée par fluorimétrie à λ_{excitation} à 330nm et λₑₘᵢₛₛᵢₒₙ à 420nm.

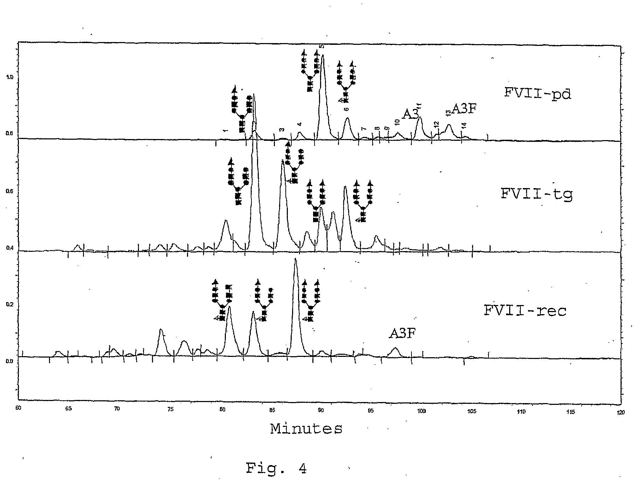

Le profil chromatographique (figure 4) du FVII-pd montre que les glycannes majoritaires sont de type biantennés, bisialylés (A2) avec un taux à 39%. On observe également en plus faible quantité des formes biantennées, bisialylées, fucosylées (A2F), monosialylées (A1) et trisialylées fucosylées et non fucosylées (A3F et A3).

L'analyse par NP-HPLC réalisée sur le FVII-tg confirme la présence d'oligosaccharides majoritairement de type A1, à un taux de 27%. Les structures A1F, A2 et A2F sont moins représentées et les formes triantennées sont présentes à l'état de traces. Cela met en évidence une différence de sialylation entre le FVII-pd et le facteur FVII-tg, moins sialylé.

### Exemple 7 : Identification par MALDI-TOFMS

La spectrométrie de masse MALDI-TOF MS (Matrix-Assisted Laser Desorption/Ionisation Time of Flight Mass Spectrometry) est une technique permettant de mesurer la masse moléculaire des peptides, protéines, glycannes, oligonucléotides, et la majorité des polymères ionisables avec une grande précision.

Les peptides, protéines et glycannes à analyser sont mélangés à une matrice qui absorbe à la longueur d'onde du laser utilisé. Les principales matrices sont l'acide α-cyano-4-hydroxycinnamique (HCCA) pour l'analyse des peptides, l'acide sinapinique (SA) pour les protéines et l'acide 2,5-dihydroxybenzoïque (DHB) pour les oligosaccharides.

La méthode consiste à irradier les co-cristaux matrice/analyte à l'aide d'un laser pulsé, ceci entraîne la désorption conjointe des molécules de matrice et d'analyte. Après ionisation en phase gazeuse, les molécules d'analyte traversent un détecteur à temps de vol. Comme les masses et temps de vol sont directement liés, la mesure de ce dernier permet la détermination de la masse de l'analyte cible. L'identification est réalisée par mesure de la masse observée, par comparaison avec la masse théorique. Le séquençage peut être effectué dans le mode MS/MS sur la base des ions fragments obtenus. L'instrument utilisé est un Bruker Autoflex 2 fonctionnant dans les modes TOF et TOF/TOF.

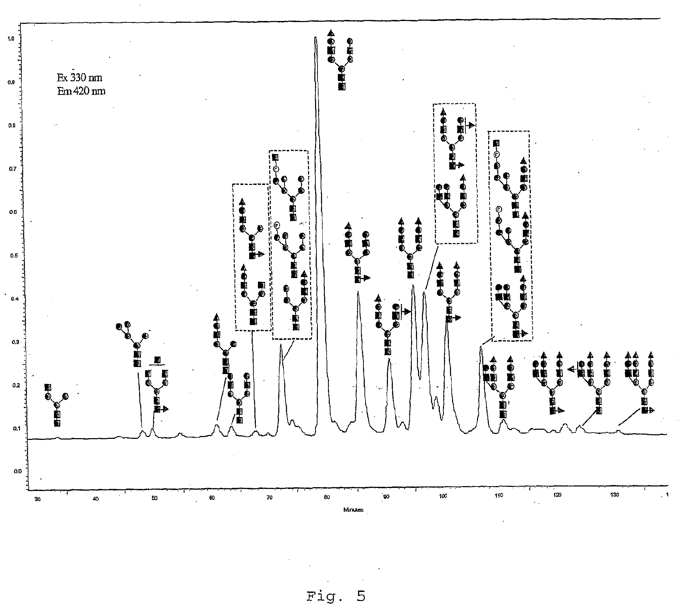

Afin d'identifier les structures glycanniques présentes dans le FVII-tg, des analyses MALDI-TOF MS ont été réalisées à partir de fractions d'élutions issus de NP-HPLC préparative (figure 5).

L'analyse MALDI-TOF du FVII-tg a permis de confirmer l'identification des glycannes séparés par NP-HPLC, à savoir des structures majoritairement monosialylées A1 et des formes minoritaires de type A1F, A2F et A2.

Cette étude a permis également d'identifier les formes minoritaires de type triantennées bisialylées et trisialylées, des formes hybrides et oligomannoses de type Man5 et Man6-P-HexNAc.

Afin d'identifier d'autres structures glycanniques, une analyse par MALDI-TOF MS du facteur FVII-tg après désialylation a été faite.

On observe après désialylation deux formes majoritaires G2 et G2F présentes respectivement à des taux de 35% et de 28% (quantification NP-HPLC). Ces résultats sont en accord avec l'identification des structures du produit « natif ».

Les autres formes identifiées sont quantitativement minoritaires et essentiellement de type oligomannose, Man6-P-HexNAc et Man7-P-HexNAc, et de type hydride. On note la présence de formes bifucosylées.

### Exemple 8 : Analyse HPCE-LIF de la liaison des acides sialiques - galactose

Concernant l'étude de la liaison acide sialique-galactose ("branching"), la procédure expérimentale est similaire à celle développée à l'Exemple 5. Après déglycosylation à la PNGaseF, les oligosaccharides sont traités par des exosialidases spécifiques de façon à assurer l'identification de la liaison et la quantification de chaque structure isolée. Les sialidases utilisées sont des enzymes recombinantes issues de *S. pneumoniae* (spécifique de la liaison α2-3, 0.02 IU, E/S=0.4 m/m), *C*. *perfringens* (spécifique des liaisons α2-3 et α2-6, 0.04 IU, E/S=0.1 m/m) et A. *urefaciens* (hydrolysant les liaisons α2-3, α2-6, α2-8 et α2-9, 0.01 IU, E/S=0.05 m/m).

Des analyses ont montré que le FVII-rec possède des structures glycaniques biantennées, sialylées, fucosylées avec des formes A2F majoritaires, et biantennées, monosialylées, fucosylées (A1F). On note des temps de migration atypiques pour ces structures A2F et A1F par rapport aux temps de migration habituellement rencontrés pour ces structures. Notamment, ces structures oligosaccharidiques sialylées présentent des temps de migration atypiques en HPCE-LIF et NP-HPLC par rapport à ceux du FVII-tg. D'autre part, l'analyse de la composition en monosaccharides n'a pas révélé d'acide sialique particulier autre que Neu5Ac et les outils de spectrométrie de masse révèlent des glycannes de masse conforme à des types bisialylés. Enfin, la desialylation des glycannes du FVII-rec permet de retrouver des comportements chromatographique et electrophorétique équivalents à ceux des oligosaccharides du FVII-tg et FVII-pd.

Ces différences de comportements électrophorétique et chromatographique peuvent donc s'expliquer par un branchement différent des acides sialiques. Cette hypothèse a été évaluée par les différentes approches d'HPCE-LIF et de MS.

Les résultats sont résumés dans le Tableau 4 ci-dessous.

**Tableau 4 : branchements des acides sialiques sur les différents lots de FVII.**

| | Sialylation (%) | α2-3 (%) | α2-6 (%) | α2-8 (%) |
|---|---|---|---|---|
| FVII-pd | 100 | 41 | 59 | 0 |
| FVII-rec | 91 | 100 | 0 | 0 |
| FVII-tg Lot C | 96 | 0 | 100 | 0 |

Les résultats montrent des isoméries distinctes au niveau des acides sialiques entre les trois FVII. En effet, les acides sialiques du FVII-rec impliquent des liaisons α2-3 alors que le FVII-tg présente des branchements α2-6 et que le FVII-pd est un mélange de ces deux isomères.

Les différences de comportements en HPCE-LIF et en NP-HPLC observés pour les glycannes du FVII-rec par rapport au FVII-tg et FVII-pd sont liées à ces différences d'isomérie au niveau des acides sialiques.

### Exemple 9 : Etude de la O-glycosylation

Le FVII comporte deux sites de O-glycosylation localisés au niveau des Ser₅₂ et Ser₆₀. Ces résidus présentent respectivement les motifs Glucose-(Xylose)₀₋₂ et Fucose. Dans le cadre de ce travail, la O-glycosylation a été étudiée par LC-ESIMS(/MS) et d'une façon similaire par MALDI-TOF(/TOF) (données non présentées).

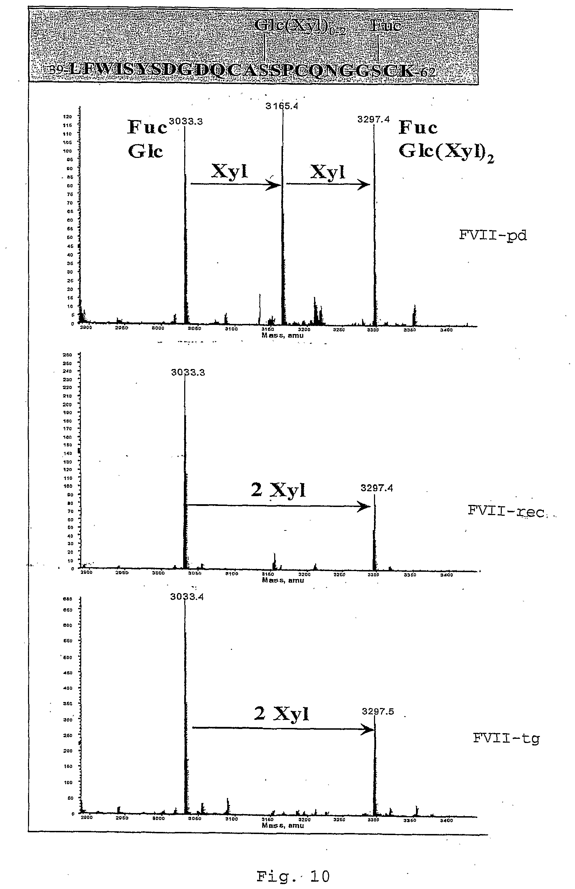

La figure 10 illustré les structures des O-glycannes des échantillons FVII-pd, FVII-rec et FVII-tg. Le pic à m/z 3297.4 correspond au peptide [Leu₃₉-Lys₆₂] comportant les motifs Glucose-(Xylose)₂ sur la Ser₅₂ et Fucose sur la Ser₆₀. Les pics à m/z 3165.4 et 3033.3 correspondent respectivement aux pertes de 1 et 2 xyloses sur ce glycopeptide. Les trois types de FVII sont uniformément fucosylés et glucosylés, mais diffèrent par le nombre de résidus xylose et la proportion des glycoformes correspondantes. Ainsi, les formes à 0, 1 et 2 xyloses sont présentes dans des proportions approximativement équivalentes dans le FVII-pd tandis que le FVII-rec et le FVII-tg comportent uniquement les formes à 0 et 2 xyloses.

L'analyse par spectrométrie de masse montre l'ion doublement chargé à m/z 1516.6 correspondant au peptide [Leu₃₉-Lys₆₂] portant les structures Fuc et Glc(Xyl)₀. Les pertes de masses successives obtenus par MS/MS, qui correspondent aux différents monosaccharides, confirment la modification des Ser₅₂ et Ser₆₀ respectivement par les résidus glucose et fucose. Le séquençage d'Edman a permis de conforter ces résultats en terme de sites modifiés.

De la même façon, l'ion doublement chargé à *m*/*z* 1648.7 correspond au peptide [Leu₃₉-Lys₆₂] portant les structures Fuc et Glc(Xyl)₂. Les différents ions fragments obtenus en MS/MS confirment que les motifs xyloses étaient liés au Glucose.

### Exemple 10 : Etude de la γ-carboxylation

Les 10 premiers acides glutamiques du FVII-pd sont γ-carboxylés, tandis que le 10^{ème} acide glutamiques du FVII-rec n'est que partiellement γ-carboxylé. Afin d'étudier la γ-carboxylation du FVII transgénique et de ses comparateurs, les peptides obtenus après digestion à l'Asp-N et à la trypsine ont été analysés par LC-MS. Les conclusions étant similaires pour ces deux enzymes, seules les données relatives à la digestion Asp-N sont présentées.

Dans ces conditions expérimentales, trois peptides [Ala₁-Lys₃₂], [Asp₃₃-Ser₄₅] et [Asp₃₃-Gly₄₇] impliquant la γ-carboxylation ont été identifiés. Le peptide [Ala₁-Lys₃₂] contient les 9 premiers acides γ-carboxyliques (Gla) alors que les deux autres peptides [Asp₃₃-Ser₄₅] et [Asp₃₃-Gly₄₇] ne contiennent que le 10^{ème} résidu Gla. Ainsi, il est plus facile d'étudier spécifiquement la modification partielle du 10^{ème} résidu Gla.

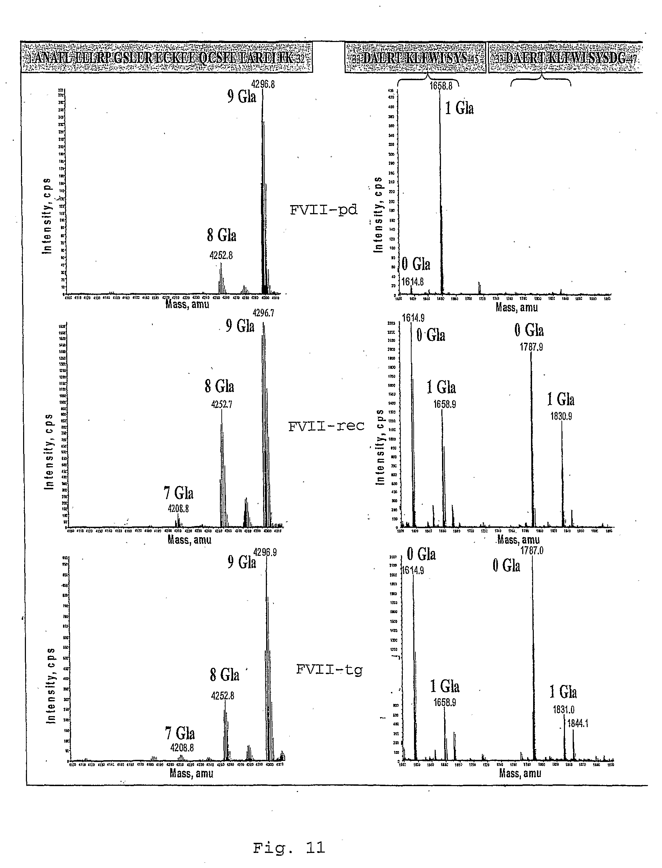

L'analyse des spectres de masse du FVII-pd (Figure 11) révèle la présence d'un pic à 4296.8 Da, majoritaire (∼90%), correspondant au peptide [Ala₁-Lys₃₂] totalement γ-carboxylé sur les 9 premiers acides glutamiques (Glu) et d'un pic à 1658.8 Da caractéristique du peptide [Asp₃₃-Ser₄₅] totalement γ-carboxylé au niveau du dixième Glu₃₅. Ces résultats montrent, en accord avec la littérature (Jurlander B. et al, Semin. Thromb. Hemost., 2001, 27, 373-384), que les 10 premiers résidus Glu du FVII-pd sont γ-carboxylés.

Les différentes données ayant été acquises dans les mêmes conditions expérimentales, une étude comparative est possible entre les différents lots de FVII (Tableau 5) .

La figure 11 montre que pour les FVII-rec et FVII-tg, le pic à 4296.8 Da correspondant au peptide [Ala₁-Lys_{32]} totalement γ-carboxylé est majoritaire. Par contre, pour les deux FVII, on note la présence majoritaire du pic à 1614.9 qui représente la caractéristique du peptide [Asp₃₃-Ser₄₅] non γ-carboxylé sur le Gla35. Ce résultat indique une γ-carboxylation partielle du 10^{ème} Gla évaluée à environ 35-40% dans le cas du FVII-rec ce qui est en accord avec la valeur publiée de ∼50% (Thim L. et al, Biochemistry, 1988, 27, 7785-7793 et Jurlander B. et al, Semin. Thromb. Hemost., 2001, 27, 373-384). Il faut également signaler la présence d'une forme à 8 Gla d'intensité non négligeable au sein du peptide [Ala₁-Lys₃₂]. Ce dernier point indique une γ-carboxylation partielle d'un ou plusieurs Gla au sein de ce peptide, la présence de "traces" d'une forme à 7 Gla soulignerait plutôt l'implication d'un seul résidu.

**Tableau 5. Résumé des données concernant la γ-carboxylation des Glu. Ces données sont issues de l'analyse par LC-ESIMS. Gla : acide γ-carboxylique.**

| | Nombre de résidus Gla | Forme majoritaire (n Gla) |
|---|---|---|
| FVII-pd | 8-10 | 10 |
| FVII-rec | 7-10 | 9 |
| FVII-tg | 7-10 | 9 |
| FVII-tg | 7-10 | 9 |

### Exemple 11 : Etude des ponts disulfure

L'étude des ponts disulfures du FVII a été réalisée à partir de l'hydrolysat trypsique obtenu dans des conditions non réductrices (conservation des ponts disulfures).

Une étape de réaction avec l'iodoacétamide a été incluse afin de bloquer d'éventuelles cystéines libres et éviter ainsi un échange de ponts disulfures durant les étapes réalisées à pH alcalin.

Dans ces conditions, on a pu confirmer l'existence de 12 ponts disulfures et apparier 10 cystéines (Tableau 6). Néanmoins, il faut souligner que les résultats obtenus relativement à cette partie de la séquence du FVII sont en accord avec l'appariement théorique. Ces conclusions sont applicables aux deux échantillons investigués : FVII-pd et FVII-tg.

**Tableau 6. Résumé des résultats obtenus quant à l'étude des ponts disulfures.**

| | FVII-pd | FVII-tg |
|---|---|---|
| PONTS DISULFURES CONFIRMES | 12 | 12 |
| Cystéines appariées | Cys₁₇ - Cys₂₂ | Cys₁₇ - Cys₂₂ |
| | Cys₁₁₄ - Cys₁₂₇ | Cys₁₁₄ - Cys₁₂₇ |
| | Cys₁₃₅ - Cys₂₆₂ | Cys₁₃₅ - Cys₂₆₂ |
| | Cys₁₅₉ - Cys₁₆₄ | Cys₁₅₉ - Cys₁₆₄ |
| | Cys₁₇₈ - Cys₁₉₄ | Cys₁₇₈ - Cys₁₉₄ |
| | Cys₃₁₀ - Cys₃₂₉ | Cys₃₁₀ - Cys₃₂₉ |
| | Cys₃₄₀ - Cys₃₆₈ | Cys₃₄₀ - Cys₃₆₈ |

La stratégie expérimentale utilisée est appliquée à un dipeptide [Gly₃₅₄-Ser₃₇₉] / [Asp₃₃₈-Lys₃₄₁] sans autre modifications post-traductionnelles (MPT) (résultats non montrés). Cette stratégie implique plusieurs approches complémentaires : i) l'identification des dipeptides contenant le ponts disulfures par un double "matching" de masses par ESI-MS et MALDI-MS entre les masses expérimentales et observées, ii) la confirmation des structures par réduction chimique des ponts S-S et identification en MS des deux peptides générés à partir du dipeptide et iii) le séquençage des dipeptides par MS/MS et microsequençage automatique d'Edman.

Les résultats montrent une première identification du dipeptide [Gly₃₅₄-Ser₃₇₉] / [Asp₃₃₈-Lys₃₄₁] impliquant un pont disulfure Cys₃₄₀-Cys₃₆₈ par « matching » de la masse expérimentale (3264.6 Da) avec la masse théorique (3264.5 Da).

Après réduction, on note la disparition du pic correspondant à ce dipeptide [Gly₃₅₄-Ser₃₇₉] / [Asp₃₃₈-Lys₃₄₁] contenant le pont Cys₃₄₀-Cys₃₆₈ au profit d'un pic à m/z 2816.3 caractéristique du peptide [Gly₃₅₄-Ser₃₇₉] (masse théorique : 2816.3 Da) contenant la Cys₃₆₈. Le séquençage par MS/MS et par la chimie d'Edman confirme l'identification du dipeptide [Gly₃₅₄-Ser₃₇₉] / [Asp₃₃₈-Lys₃₄₁] et du pont disulfure Cys₃₄₀-Cys₃₆₈.

### Exemple 12 : Etude de la β-Hydroxylation de l'acide aspartique 63

L'analyse des peptides est réalisée à partir d'un « digest » trypsique de la protéine selon des méthodes connues de l'homme du métier. Les peptides sont analysés en MALDI-MS. L'identification des peptides hydroxylés est réalisée par mesure de la masse observée, par comparaison avec la masse théorique. L'instrument utilisé est un Bruker Autoflex 2 fonctionnant dans le mode TOF.

Les différents résultats obtenus sont présentés dans le Tableau 7. Il faut souligner que la littérature (Thim L. et al, Biochemistry, 1988, 27, 7785-7793) rapporte l'absence de β-hydroxylation des FVII-pd et FVII-rec, alors qu'il a été observé une modification partielle de ces deux échantillons, cependant de façon moins abondante que les différents lots de FVII-tg.

**Tableau 7. Résumé des données concernant l'hydroxylation de l'Asp₆₃. Ces données sont issues de l'analyse par LC-ESIMS des peptides trypsiques. (*) β-hydroxylation moins "abondante" que dans les FVII-tg. (**) Valeur issue de la littérature (Thim L. et al, Biochemistry, 1988, 27, 7785-7793.**

| | Hydroxylation Asp₆₃ |
|---|---|
| FVII-pd | Partielle * |
| FVII-rec | Non ** |
| FVII-tg | Partielle |
| FVII-tg | Partielle |
| FVII-tg | Partielle |

### Exemple 13 : Etude de la présence des structures Galα1, 3Gal

Afin d'étudier la présence éventuelle des structures Galα1,3Gal dans les FVII-tg, on utilise comme témoin positif la thyroglobuline bovine contenant des structures Galα1,3Gal.

Les produits sont, après déglycosylation par la PNGase F, traités par des sialidases, fucosidases et α-galactosidases spécifiques des branchements Galα1,3Gal.

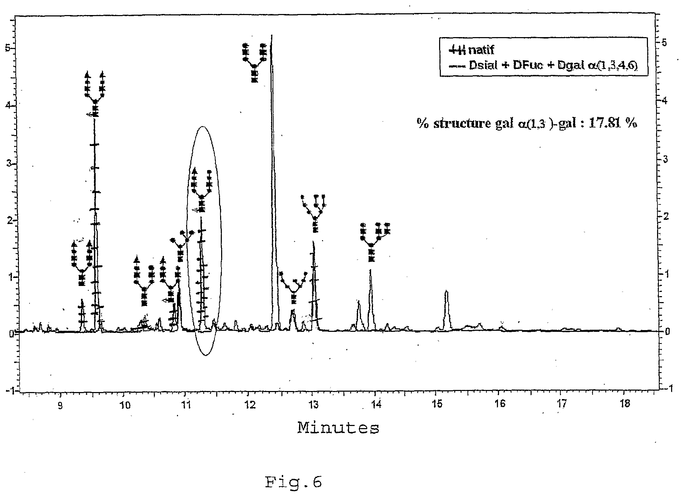

La figure 6 présente l'étude réalisée sur la thyroglobuline bovine avec la superposition du profil natif du FVII et du profil désialylé, défucosylé et α-dégalactosylé (Dsial+DFuc+Dgalα (1, 3, 4, 6)). Le profil glycanique obtenu après α-dégalactosylation montre que cette structure disparaît totalement après traitement par l'enzyme prouvant ainsi que α-galactosidase utilisée est parfaitement active. Le taux de structure Galα1,3Gal obtenu de 17.8% est cohérent avec le taux attendu.

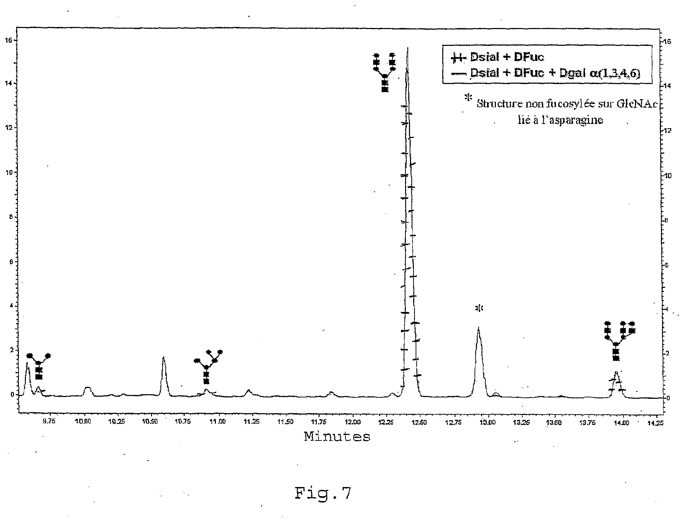

La figure 7 présente la superposition des électrophorégrammes du produit désialylé et défucosylé du FVII-tg (Dsial+DFuc) avec le produit désialylé, défucosylé et α-dégalactosylé (Dsial+DFuc+Dgalα(1, 3, 4, 6)).

On observe que les deux profils sont parfaitement superposables. Ces résultats suggèrent l'absence de structures Galα1,3Gal dans le FVII-tg. On note la présence de structures minoritaires (*) correspondant à des glycannes dont le fucose n'est pas en position proximale.

### Exemple 14 : Quantification des motifs saccharidiques Galα1,3Gal

10 µg des FVII humain, recombinant et transgénique ont été traités en conditions de réduction des ponts disulfures, et déposés sur gel d'électrophorèse et transférés sur une membrane de nitrocellulose. Un échantillon de thyroglobuline bovine (Sigma, T1001, Thyroglobulin from bovin thyroid) est utilisé comme contrôle positif du fait de sa forte teneur en épitope alpha(1,3) galactosyl. La présence du motif α-Gal (Galactose-alphal-3Galactose-(3)4GlcNAc-R) est révélée par la lectine purifiée *Marasmius oreades* agglutinin (EY laboratoiries, H-9001, MOA-HRP) marquée à la peroxydase et spécifique de l'épitope α-Gal. L'activité peroxydasique est ensuite détectée par un substrat chromogénique (Fluka, 4-chloro-1-naphtol) et quantifiée par numérisation du signal (Bio-Rad, Quantity-one). Une membrane identique est traitée avec une alpha-galactosidase (Prozyme, Green coffee bean) de façon à confirmer la spécificité du signal. En parallèle, la quantité de protéine totale est également estimée par coloration d'un gel SDS-PAGE identique, coloré au bleu de Coomassie.

Les résultats montrent la spécificité la lectine *Marasmius oreades* (MOA) du fait du fort signal observé pour la thyroglobuline bovine et de sa disparition suivant le traitement avec une alpha-galactosidase. Les résultats exprimés en rapport d'intensité de signal MOA divisée par l'intensité du signal correspondant à la quantité de protéine analysée démontrent un niveau de signal α-Gal résiduel (2,3 à 3,7) pour l'échantillon de FVII-tg alors que le FVII recombinant présente une valeur supérieure.

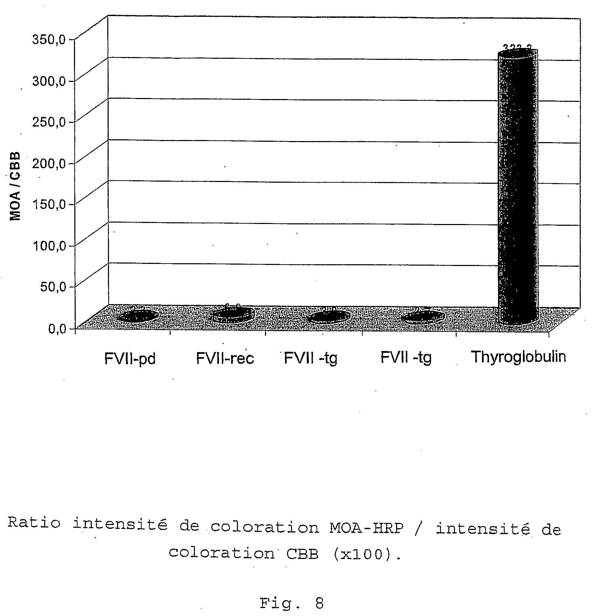

Ces résultats (cf figure 8) démontrent l'absence ou la très faible prévalence de l'épitope α-Gal sur le FVII transgénique exprimé chez le lapin, comparativement à un FVII recombinant produit par des cellules de hamster par exemple.

Le Tableau A résume les étapes du procédé selon une mise en oeuvre préférée de l'invention pour fournir la composition de FVII de l'invention, et fournit les différents rendements, la pureté et les activités spécifiques obtenus à chaque étape.

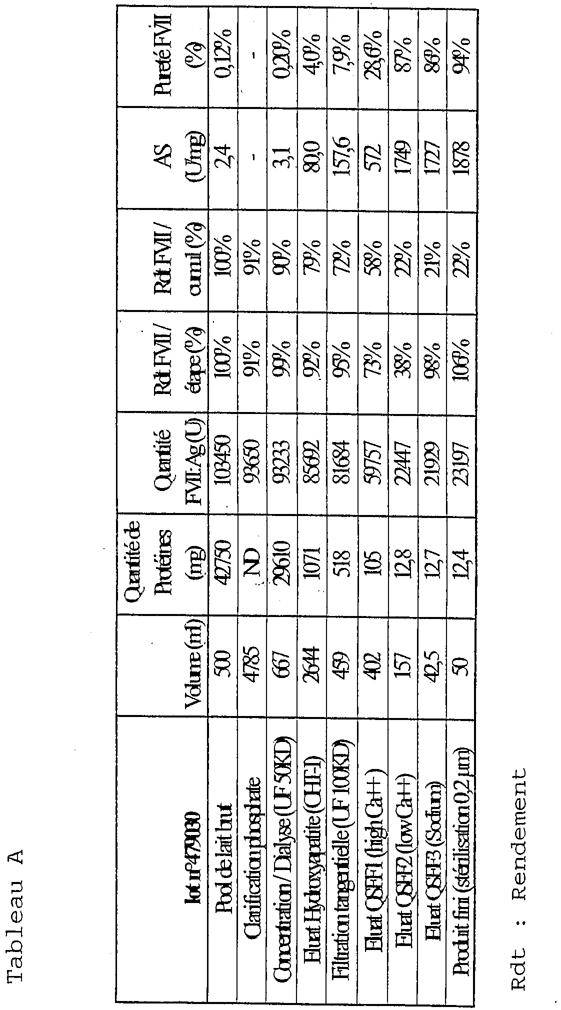

### SEQUENCE LISTING

<110> Laboratoire Français du Fractionnement et des Biotechnologies
<120> FVII transgénique
<130> LFB
<160> 1
<170> PatentIn version 3.1
<210> 1
   <211> 406
   <212> PRT
   <213> Homo sapiens
<400> 1

## Revendications

1. Composition de facteur VII (FVII) transgénique, chaque molécule de facteur VII de la composition présentant deux sites de N-glycosylation, **caractérisée en ce que** :
- parmi toutes les molécules de FVII de la composition, le taux de motifs glycanniques Galα1,3Gal est compris entre 0 et 4%, le taux de de motifs glycanniques Galα1,3Gal étant mesuré par lectine-blot avec révélation au 4-chloro-1-naphtol, et
- **en ce que** tous les acides sialiques du facteur VII impliquent des liaisons α2-6, et
- **en ce que** le facteur VII de la composition est produit par des lapines transgéniques.

2. Composition selon la revendication 1, **caractérisée en ce que**, parmi tous les motifs glycanniques du facteur VII de la composition, au moins 40% sont des formes glycanniques biantennées, monosialylées, et dans laquelle les formes glycanniques biantennées, monosialylées du facteur VII sont majoritaires.

3. Composition selon la revendication 2, **caractérisée en ce que**, lorsque les formes glycanniques biantennées monosialylées du facteur VII sont majoritaires, parmi les formes glycanniques biantennées, monosialylées du facteur VII, les formes glycanniques majoritaires sont non fucosylées, et le taux de fucosylation du facteur VII est compris entre 20% et 50%.

4. Composition selon l'une quelconque des revendications 1 à 3, **caractérisé en ce que** le facteur VII est activé.

5. Composition de facteur VII selon l'une quelconque des revendications 1 à 4, pour son utilisation comme médicament.

6. Utilisation d'une composition facteur VII selon l'une quelconque des revendications 1 à 4, pour la préparation d'un médicament destiné au traitement des patients atteints d'hémophilie, au traitement des traumatismes hémorragiques multiples, au traitement des saignements massifs non contrôlables par une hémostase chirurgicale, ou au traitement des hémorragies dues à un surdosage en anticoagulants.

7. Composition pharmaceutique comprenant un facteur VII tel que défini selon l'une quelconque des revendications 1 à 4, et un excipient et/ou un véhicule pharmaceutiquement acceptables.

8. Procédé d'extraction et de purification de FVII transgénique, contenu dans du lait d'une lapine transgénique, comprenant les étapes suivantes consistant à :
a) extraire le FVII à partir du lait, le facteur VII étant lié aux sels et/ou complexes organiques et/ou inorganiques de calcium dudit lait, par la précipitation de composés de calcium obtenue par ajout dans le lait d'un sel soluble, dont l'anion est choisi pour son aptitude à former lesdits composés de calcium insolubles pour ainsi libérer le facteur VII desdits sels et/ou complexes, le facteur VII étant ainsi présent dans une phase liquide,
b) séparer la phase liquide enrichie en la protéine du précipité de composés de calcium, ladite phase liquide étant en outre séparée en une phase lipidique et une phase aqueuse non lipidique comprenant la protéine,
c) soumettre la phase aqueuse non lipidique à une étape de chromatographie d'affinité, en utilisant un tampon d'élution à base d'un sel de phosphate à une concentration prédéterminée, et
d) soumettre l'éluât de facteur VII, obtenu selon l'étape c), à deux ou trois étapes de chromatographie sur des colonnes échangeuses d'anions de type base faible utilisant des tampons appropriés pour des élutions successives du facteur VII retenu sur lesdites colonnes.

9. Procédé selon la revendication 8, dans lequel le sel soluble est un sel de phosphate choisi dans le groupe constitué par le phosphate de sodium, le phosphate de lithium, le phosphate de potassium, le phosphate de rubidium et le phosphate de césium, et est, en particulier, le phosphate de sodium, ou dans lequel le sel soluble est un oxalate d'un métal alcalin, en particulier l'oxalate de sodium ou de potassium, ou un carbonate d'un métal alcalin, en particulier le carbonate de sodium ou de potassium, ou leur mélange.

10. Procédé selon l'une des revendications 8 à 9, dans lequel la concentration du sel soluble en solution aqueuse est comprise entre 100 mM et 3 M, de façon plus préférée, entre 200 mM et 500 mM et, en particulier, entre 200 mM et 300 mM.

11. Procédé selon l'une des revendications 8 à 10, dans lequel dans lequel l'étape b) est effectuée par centrifugation.

12. Procédé selon l'une des revendications 8 à 11, comprenant, après l'étape de séparation, une étape de filtration de la phase aqueuse non lipidique effectuée successivement sur des filtres de porosité décroissante, de préférence de 1 µm puis de 0,45 µm, suivie d'une étape de concentration/dialyse par ultrafiltration, et comprenant optionnellement une étape de filtration de la phase liquide à travers des filtres de porosité décroissante, de préférence de 1 µm puis de 0,45 µm.

13. Procédé selon l'une des revendications 8 à 12, dans lequel la chromatographie d'affinité est mise en oeuvre sur une colonne chromatographique dont le support est un gel d'hydroxyapatite (Ca₁₀(PO₄)₆(OH)₂) ou un gel de fluoroapatite (Ca₁₀(PO₄)₆F₂), et le tampon d'élution est un tampon à base de phosphate de sodium 0,25 M-0,35 M, pH 7,5-8,5.

14. Procédé selon l'une des revendications 8 à 13, dans lequel l'éluât de FVII obtenu à l'issue de l'étape c) est ensuite soumis à une filtration tangentielle.

15. Procédé selon l'une des revendications 8 à 14, dans lequel, la première étape chromatographique est mise en oeuvre par un support chromatographique de type gel Q-Sepharose FF et dans lequel l'élution du FVII est effectuée par un tampon aqueux à base de Tris 0,05 M et de chlorure de calcium 0,020 M-0,05 M, pH 7,0-8,0.

16. Procédé selon l'une des revendications 8 à 15, dans lequel, la deuxième étape chromatographique est mise en oeuvre par un support chromatographique de type gel Q-Sepharose FF sur lequel est injecté le premier éluat de FVII, obtenu par la première étape chromatographique sur échangeur d'anions, et dans lequel l'élution du FVII est effectuée par un tampon aqueux à base de Tris 0,05 M et de chlorure de calcium 0,005 M, pH 7,0-8,0.

17. Procédé selon l'une des revendications 8 à 16, dans lequel la troisième étape chromatographique est mise en oeuvre par un support chromatographique de type gel Q-Sepharose FF sur lequel est injecté le deuxième éluat de FVIII, obtenu par la deuxième étape chromatographique sur échangeur d'anions, et dans lequel l'élution du FVII est effectuée par un tampon aqueux à base de Tris 0,02 M et de chlorure de sodium 0,20-0,30 M, de préférence 0,28 M, pH 6,5-7,5.

18. Procédé selon l'une des revendications 8 à 17, comprenant au moins l'une des étapes suivantes de formulation, inactivation virale et stérilisation.

19. Procédé selon l'une des revendications 8 à 18, comprenant, avant l'étape de chromatographie d'affinité, une étape de traitement anti-viral qui est effectuée par solvant/détergent.

20. Procédé selon l'une des revendications 8 à 19, dans lequel l'éluat issu de la deuxième étape chromatographique sur échangeur d'anions est soumis à une étape de nanofiltration.

## Patentansprüche

1. Transgene Faktor VII (FVII)-Zusammensetzung, wobei jedes Faktor VII-Molekül der Zusammensetzung zwei N-Glykosylierungsstellen aufweist, **dadurch gekennzeichnet, dass**:
- unter allen FVII-Molekülen der Zusammensetzung, der Anteil an Galα1,3Gal-Glycanmustern zwischen 0 und 4 % liegt, wobei der Anteil an Galα1,3Gal-Glycanmustern mit Lectin-Blot und Nachweis mit 4-Chlor-1-Naphthol gemessen wird, und
- dadurch, dass alle Faktor VII-Sialinsäuren α2-6-Bindungen beinhalten, und
- dadurch, dass der Faktor VII der Zusammensetzung durch transgene Zibben hergestellt wird.

2. Zusammensetzung nach Anspruch 1, **dadurch gekennzeichnet, dass** mindestens 40 % aller Glycanmuster des Faktors VII der Zusammensetzung biantennäre, monosialylierte Glycanstrukturen sind, und wobei die biantennären, monosialylierten Glycanstrukturen des Faktors VII überwiegen.

3. Zusammensetzung nach Anspruch 2, **dadurch gekennzeichnet, dass** unter den biantennären, monosialylierten Glycanstrukturen des Faktors VII, wenn die biantennären, monosialylierten Glycanstrukturen des Faktors VII überwiegen, die überwiegenden Glycanstrukturen nicht fucosyliert sind, und die Fucosylierungsrate des Faktors VII zwischen 20 % und 50 % liegt.

4. Zusammensetzung nach einem der Ansprüche 1 bis 3, **dadurch gekennzeichnet, dass** der Faktor VII aktiviert ist.

5. Faktor VII-Zusammensetzung nach einem der Ansprüche 1 bis 4 für seine Verwendung als Medikament.

6. Verwendung einer Faktor VII-Zusammensetzung nach einem der Ansprüche 1 bis 4 für die Herstellung eines Medikaments zur Behandlung der Hämophilie-Patienten, zur Behandlung der hämorrhagischen Polytraumata, zur Behandlung der starken Blutungen, die nicht durch eine chirurgische Hämostase unter Kontrolle zu bringen sind, oder zur Behandlung der Blutungen wegen einer Gerinnungshemmer-Überdosierung.

7. Pharmazeutische Zusammensetzung, umfassend einen Faktor VII nach einem der Ansprüche 1 bis 4 und einen pharmazeutisch annehmbaren Hilfsstoff und/oder einen pharmazeutisch annehmbaren Arzneistoffträger.

8. Verfahren zur Extraktion und Reinigung von in der Milch einer transgenen Zibbe enthaltenem, transgenem FVII, folgende Schritte umfassend:
a) Extraktion des FVII aus der Milch, wobei der Faktor VII mit den Salzen und/oder organischen Calcium-Komplexen und/oder den anorganischen Calcium-Komplexen der genannten Milch gebunden ist, durch das Abscheiden von Calciumverbindungen, das durch Zufügung eines löslichen Salzes in die Milch, dessen Anion für seine Fähigkeit zur Bildung der genannten unlöslichen Calciumverbindungen gewählt wird, um somit den Faktor VII aus den genannten Salzen und/oder Komplexen freizusetzen, wobei der Faktor VII damit in einer Flüssigphase vorhanden ist,
b) Trennung der Flüssigphase, die mit dem Protein der gefällten Calciumverbindungen angereichert ist, wobei die genannte Flüssigphase außerdem in eine Lipidphase und eine nicht lipidhaltige, das Protein enthaltende Wasserphase getrennt wird,
c) Unterziehen der nicht lipidhaltigen Wasserphase einem Schritt der Affinitäts-Chromatographie und dabei Verwendung eines Elutionspuffers auf Basis eines Phosphatsalzes zu einer vorbestimmten Konzentration, und
d) Durchlaufen des beim Schritt c) gewonnenen Faktor VII-Eluats von zwei oder drei Chromatographiestufen an schwach basischen Anionentauschkolonnen unter Verwendung von Puffern, die für aufeinander folgende Elutionen des auf den genannten Kolonnen zurückgehaltenen Faktors VII geeignet sind.

9. Verfahren nach Anspruch 8, wobei das lösliche Salz ein Phosphatsalz ausgewählt aus der Gruppe bestehend aus Natriumphosphat, Lithiumphosphat, Kaliumphosphat, Rubidiumphosphat und Caesiumphosphat und im Besonderen Natriumphosphat ist, oder wobei das lösliche Salz ein Oxalat eines Alkalimetalls, im Besonderen Natrium- oder Kaliumoxalat, oder ein Karbonat eines Alkalimetalls, im Besonderen Natrium- oder Kaliumcarbonat, oder deren Mischung ist.

10. Verfahren nach einem der Ansprüche 8 bis 9, wobei die Konzentration des löslichen Salzes in Wasserlösung zwischen 100 mM und 3 M, bevorzugterweise zwischen 200 mM und 500 mM und im Besonderen zwischen 200 mM und 300 mM liegt.

11. Verfahren nach einem der Ansprüche 8 bis 10, wobei Schritt b) durch Zentrifugation erfolgt.

12. Verfahren nach einem der Ansprüche 8 bis 11, umfassend nach dem Schritt der Trennung einen Schritt zur Filtration der nicht lipidhaltigen Wasserphase, welcher nach einander auf Filtern mit abnehmender Porosität, vorzugsweise von 1 µm dann 0,45 µm erfolgt, gefolgt von einem Schritt zur Konzentration/Dialyse durch Ultrafiltration, und optional umfassend einen Schritt zur Filtration der Wasserphase durch Filter mit abnehmender Porosität, vorzugsweise von 1 µm dann 0,45 µm, hindurch.

13. Verfahren nach einem der Ansprüche 8 bis 12, wobei die Affinitätschromatographie auf einer chromatographischen Säule durchgeführt wird, deren Träger ein Hydroxyapatit-Gel (Ca₁₀(PO₄)₆(OH)₂) oder ein Fluorapatit-Gel (Ca₁₀(PO₄)₆F₂) ist, und der Elutionspuffer ein Puffer auf Basis von Natriumphosphat 0,25 M - 0,35 M, pH 7,5 - 8,5 ist.

14. Verfahren nach einem der Ansprüche 8 bis 13, wobei das FVII-Eluat, das am Ende des Schritts c) gewonnen wird, anschließend einer tangentialen Filtration unterzogen wird.

15. Verfahren nach einem der Ansprüche 8 bis 14, wobei der erste chromatographische Schritt über einen chromatographischen Träger vom Typ Q-Sepharose FF-Gel durchgeführt wird und wobei die Elution des FVII über einen wässrigen Puffer auf Basis von Tris 0,05 M und von Calciumchlorid 0,020 M - 0,05 M, pH 7,0 - 8,0 erfolgt.

16. Verfahren nach einem der Ansprüche 8 bis 15, wobei der zweite chromatographische Schritt über einen chromatographischen Träger vom Typ Q-Sepharose FF-Gel durchgeführt wird, auf welchen das erste, bei dem ersten chromatographischen Schritt auf Anionaustauscher gewonnene FVII-Eluat eingespritzt wird, und wobei die Elution des FVII durch einen wässrigen Puffer auf Basis von Tris 0,05 M und Calciumchlorid 0,005 M, pH 7,0 - 8,0 erfolgt.

17. Verfahren nach einem der Ansprüche 8 bis 16, wobei der dritte chromatographische Schritt über einen chromatographischen Träger vom Typ Q-Sepharose FF-Gel durchgeführt wird, auf welchen das zweite, bei dem zweiten chromatographischen Schritt auf Anionaustauscher gewonnene FVII-Eluat eingespritzt wird, und wobei die Elution des FVIII durch einen wässrigen Puffer auf Basis von Tris 0,02 M und Natriumchlorid 0,20 - 0,30 M, vorzugsweise 0,28 M, pH 6,5 - 7,5 erfolgt.

18. Verfahren nach einem der Ansprüche 8 bis 17, mindestens einen der folgenden Schritte zur Formulierung, viralen Inaktivierung und Sterilisation umfassend.

19. Verfahren nach einem der Ansprüche 8 bis 18, vor dem Schritt der Affinitätschromatographie einen Schritt zur antiviralen Behandlung umfassend, welcher mit Hilfe eines Lösungsmittels/Reinigungsmittels erfolgt.

20. Verfahren nach einem der Ansprüche 8 bis 19, bei welchem das aus dem zweiten chromatographischen Schritt auf Anionaustauscher gewonnene Eluat einem Schritt zur Nanofiltration unterzogen wird.

## Claims

1. A composition of transgenic factor VII (FVII), each molecule of factor VII of the composition exhibiting two N-glycosylation sites, **characterized in that**:
- among all the molecules of FVII of the composition, the rate of Galα1,3Gal glycan moieties is comprised between 0 and 4%, the rate of Galα1,3Gal glycan moieties being measured by lectin-blot with exposure to 4-chloro-1-naphtol, and
- all of the sialic acids of the FVII imply α2-6 links, and
- factor VII of the composition is produced by transgenic female rabbits.

2. A composition according to claim 1, **characterized in that** among all the glycan moieties of the factor VII of the composition, at least 40% are biantennary, monosialylated, glycan forms, and wherein the biantennary, monosialylated, glycan forms of the FVII are in majority.

3. A composition according to claim 2, **characterized in that** wherein the biantennary, monosialylated, glycan forms of the factor VII are in majority, among the biantennary, monosialylated glycan forms of the factor VII, the majority of glycan forms are non-fucosylated and the rate of fucosylation of factor VII is comprised between 20% and 50%.

4. A composition according to anyone of claims 1 to 3, wherein the factor VII is activated.

5. A composition of factor VII according to anyone of claims 1 to 4, for use as medicament.

6. A use of a composition of factor VII according to any one of claims 1 to 4, for preparing a medicament intended to the treatment of patients suffering of haemophilia, to the treatment of multiple hemorrhagic traumas, to the treatment of incontrollable massive bleedings by surgical haemostasis or to the treatment of bleedings or hemorrages due to an overdose of anticoagulants.

7. A pharmaceutical composition comprising a factor VII as defined according to anyone of claims 1 to 4, and a pharmaceutically acceptable excipient and/or carrier.

8. A process of extraction and of purification of transgenic FVII, contained in the milk of a transgenic female rabbit, including the following steps consisting of:
a) extracting the FVII from the milk, the factor VII being bound to organic and/or inorganic salts and/or complexes of calcium of the said milk, by precipitation of calcium compounds obtained by addition to the milk of a soluble salt, the anion of which is selected for its capability to form said insoluble calcium compounds in order to release in this way the factor VII from said salts and/or complexes, the factor VII being then present in a liquid phase,
b) separating the protein-enriched liquid phase from the precipitate of calcium compounds, said liquid phase being further separated in a lipidic phase and an aqueous nonlipidic phase containing the protein,
c) subjecting the aqueous nonlipidic phase to an affinity chromatography step, using an elution buffer based on a phosphate salt in a predetermined concentration, and
d) subjecting the eluate of factor VII, obtained according to the step c), to two or three chromatography steps on weak basic type anion exchange columns using buffers suitable for successive elutions of the factor VII retained on said columns.

9. The process according to claim 8, wherein the soluble salt is a phosphate salt, selected from the group consisting of sodium phosphate, lithium phosphate, potassium phosphate, rubidium phosphate and cesium phosphate, and is, in particular, sodium phosphate or wherein the soluble salt is an alcali metal oxalate, particularly sodium or potassium oxalate, or an alcali metal carbonate, in particular, sodium or potassium carbonate, or a mixture thereof.

10. The process according to any one of claims 8 to 9, wherein the concentration of the soluble salt in aqueous solution is comprised between 100 mM and 3 M, more preferably between 200 mM and 500 mM and, in particular, between 200 mM and 300 mM.

11. The process according to any one of claims 8 to 10, wherein the step b) is carried out by centrifugation.

12. The process according to any one of claims 8 to 11, including, after the separation step, a step of filtration of the nonlipidic aqueous phase carried out successively on filters with a decreasing porosity, preferably of 1 µm then of 0,45 µm, followed by a step of concentration/dialysis by ultrafiltration, and optionally comprising a filtration step of the lipidic phase through filters with a decreasing porosity, preferably of 1 µm, then of 0,45 µm.

13. The process according to any one of claims 8 to 12, wherein the affinity chromatography is carried out on a chromatographic column, the support of which is a hydroxyapatite gel (Ca₁₀(PO₄)₆(OH₂)) or a fluoroapatite gel (Ca₁₀(PO₄)₆F₂), and the elution buffer is a buffer based on 0,25 M-0,35 M sodium phosphate, pH 7,5-8,5.

14. The process according to any one of claims 8 to 13, wherein the eluate of FVII resulting from the step c) is subsequently subjected to a tangential filtration.

15. The process according to anyone of claims 8 to 14, wherein the first chromatographic step is carried out on a Q-Sepharose® FF gel type chromatographic support and wherein the elution of FVII is carried out with an aqueous buffer based on 0,05 M Tris and 0,020 M-0,05 M calcium chloride, pH 7,0-8,0.

16. The process according to any one of claims 8 to 15, wherein, the second chromatographic step is carried out on a Q-Sepharose® FF gel type chromatographic support onto which is injected the first eluate of FVII obtained in the first anion exchange chromatography step and wherein the elution of FVII is carried out with an aqueous buffer based on 0,05 M Tris and 0,005 M calcium chloride, pH 7,0-8,0.

17. The process according to any one of claims 8 to 16, wherein the third chromatographic step is carried out on Q-Sepharose® FF gel type chromatographic support onto which is injected the second eluate of FVII, obtained in the second anion exchange chromatography step and wherein the elution of FVII is carried out with an aqueous buffer based on 0,02 M Tris and 0,20-0,30 M, preferably 0,28 M, sodium chloride, pH 6,5-7,5.

18. The process according to any one of claims 8 to 17, comprising at least one of the following steps of formulation, virus inactivation and sterilization.

19. The process according to any one of claims 8 to 18, comprising, prior to the affinity chromatography step, an antiviral treatment step, carried out by solvent/detergent.

20. The process according to anyone of claims 8 to 19, wherein the eluate resulting from the second anion exchange chromatography step is subjected to a nanofiltration step.
